(19) Europäisches Patentamt
European Patent Office
Office européen des brevets

(11) Veröffentlichungsnummer: **0 373 472 B1**

# (12) EUROPÄISCHE PATENTSCHRIFT

(45) Veröffentlichungstag der Patentschrift: **08.06.94**

(51) Int. Cl.5: **C07D 239/47**, A01N 47/36, C07D 239/52, C07D 251/42, C07D 251/46

(21) Anmeldenummer: **89122350.5**

(22) Anmeldetag: **04.12.89**

Die Akte enthält technische Angaben, die nach dem Eingang der Anmeldung eingereicht wurden und die nicht in dieser Patentschrift enthalten sind.

## (54) Substituierte Sulfonylharnstoffe.

(30) Priorität: **15.12.88 DE 3842177**

(43) Veröffentlichungstag der Anmeldung: **20.06.90 Patentblatt 90/25**

(45) Bekanntmachung des Hinweises auf die Patenterteilung: **08.06.94 Patentblatt 94/23**

(84) Benannte Vertragsstaaten: **BE CH DE ES FR GB IT LI**

(56) Entgegenhaltungen:
**EP-A- 0 162 723**
**EP-A- 0 169 815**
**EP-A- 0 205 348**

**CHEMICAL ABSTRACTS, Band 99, 1983, Seite 647, Zusammenfassung Nr. 212540v, Columbus, Ohio, US**

(73) Patentinhaber: **BASF Aktiengesellschaft**
**Carl-Bosch-Strasse 38**
**D-67063 Ludwigshafen(DE)**

(72) Erfinder: **Mayer, Horst, Dr.**
**Faselwiese 19**
**D-6700 Ludwigshafen(DE)**
Erfinder: **Hamprecht, Gerhard, Dr.**
**Rote-Turm-Strasse 28**
**D-6940 Weinheim(DE)**
Erfinder: **Wuerzer, Bruno, Dr.**
**Ruedigerstrasse 13**
**D-6701 Otterstadt(DE)**
Erfinder: **Westphalen, Karl-Otto, Dr.**
**Mausbergweg 58**
**D-6720 Speyer(DE)**

EP 0 373 472 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte Sulfonylharnstoffe der allgemeinen Formel I

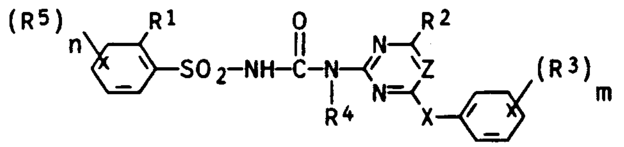

I

in der die Substituenten und die Indices folgende Bedeutung haben:

X Sauerstoff oder Schwefel;

Z Stickstoff oder eine Methingruppe = CH-;

$R^1$ Halogen, $C_1$-$C_4$-Alkoxycarbonyl, welches ein bis drei der folgenden Reste tragen kann: Halogen, $C_1$-$C_4$-Alkoxy, $C_1$-$C_4$-Halogenalkoxy, $C_1$-$C_4$-Alkylthio und/oder $C_1$-$C_4$-Halogenalkylthio; $C_1$-$C_3$-Alkoxy, welches ein bis drei der folgenden Reste tragen kann: Halogen, $C_1$-$C_4$-Alkoxy, $C_1$-$C_4$-Halogenalkoxy, $C_1$-$C_4$-Alkylthio und/oder $C_1$-$C_4$-Halogenalkylthio; oder ein Rest -CONR$^6$R$^7$, worin

$R^6$ Wasserstoff, eine $C_1$-$C_8$-Alkylgruppe oder eine $C_1$-$C_6$-Alkoxygruppe und

$R^7$ Wasserstoff oder eine $C_1$-$C_8$-Alkylgruppe bedeuten;

$R^2$ Halogen; $C_1$-$C_4$-Alkoxy, oder $C_1$-$C_4$-Alkyl, welche ein bis drei der folgenden Reste tragen können: Halogen, $C_1$-$C_4$-Alkoxy und/oder $C_1$-$C_4$-Alkylthio;

$R^3$ $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio, Halogen, Cyano, Nitro, Amino, Mono-$C_1$-$C_4$-alkylamino, Di-$C_1$-$C_4$-alkylamino, $C_2$-$C_6$-Alkenyl, $C_1$-$C_4$-Alkoxycarbonyl, $C_1$-$C_4$-Alkanoyl oder Benzyl; ein 5- oder 6-gliedriger über sein Stickstoffatom gebundener gesättigter Heterocyclus, enthaltend neben Methylengruppen ein Stickstoffatom, wobei dieser Cyclus außerdem ein Sauerstoff- oder ein Schwefelatom enthalten kann,
sowie an benachbarte Ringpositionen gebundenes -OCRR'O-, wobei R und R' Wasserstoff oder $C_1$-$C_4$-Alkyl bedeuten;

$R^4$ Wasserstoff oder $C_1$-$C_4$-Alkyl;

$R^5$ Halogen, $C_1$-$C_4$-Alkyl, $C_1$-$C_4$-Alkoxy, $C_1$-$C_4$-Alkylthio oder $C_1$-$C_4$-Halogenalkyl;

m 0 bis 3, oder 0 bis 5, wenn $R^3$ Halogen bedeutet, wobei die Reste $R^3$ verschieden sein können, wenn m 2 oder 3 bedeutet;

n 0 bis 2, wobei die Reste $R^5$ verschieden sein können, wenn n 2 bedeutet;

sowie deren umweltverträgliche Salze.

Desweiteren betrifft die Erfindung Verfahren zur Herstellung der Verbindungen I sowie ihre Verwendung als Herbizide.

Aus der JP-A 58 126 872 sind Pyrimidyl- und Triazinylsulfonylharnstoffe der Formel I'

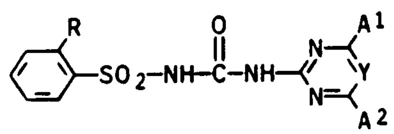

I'

bekannt, in denen die Reste u.a. folgende Bedeutung haben:

R : ggf. substituiertes Phenyl oder Phenoxy

Y : Stickstoff oder die Methingruppe

$A^1$, $A^2$ : unabhängig voneinander Wasserstoff, Halogen, Alkylgruppe; Alkoxygruppe, Halogenalkylgruppe, Alkylthioalkylgruppe, Phenylthiogruppe und/oder Phenoxygruppe.

Diese Verbindungen lassen jedoch wegen der geringen Selektivität gegen Schadpflanzen sowie wegen der relativ hohen Aufwandmengen als Herbizide zu wünschen übrig.

Der Erfindung lag daher die Aufgabe zugrunde, neue Verbindungen aus der Klasse der substituierten Sulfonylharnstoffe mit verbesserten herbiziden Eigenschaften zu finden.

Entsprechend dieser Aufgabe wurden die eingangs definierten substituierten Sulfonylharnstoffe I gefunden.

Im nachstehenden Reaktionsschema über die Verfahren zur Herstellung von I sind zur besseren Übersichtlichkeit die Reste

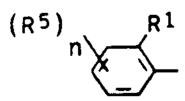 durch $\Phi^1$ und 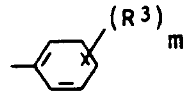 durch $\Phi^2$

ersetzt.

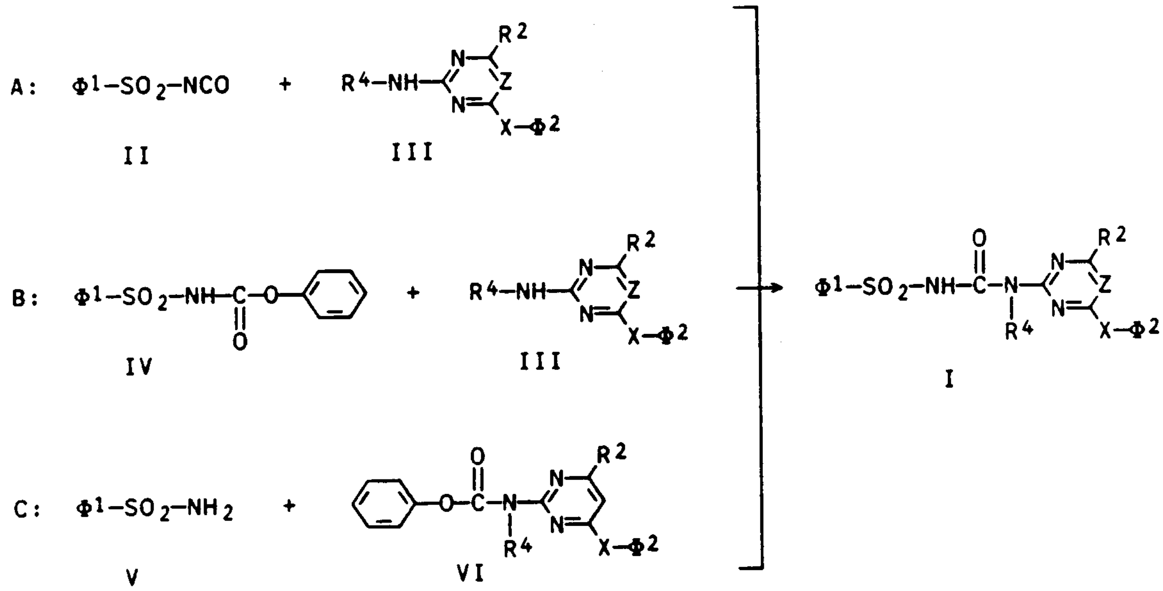


Im einzelnen gelten für diese Verfahren die folgenden Bedingungen:

A: Man setzt ein Sulfonylisocyanat II in an sich bekannter Weise (EP-A 7687) in einem inerten organischen Lösungsmittel in Anwesenheit oder Abwesenheit einer Base mit ungefähr der stöchiometrischen Menge eines 2-Amino-heteroarylethers III bei 0 bis 120 °C, vorzugsweise 10 bis 100 °C um.
Bevorzugt führt man diese Umsetzung in Acetonitril, Toluol oder Methylenchlorid in Anwesenheit von 0 bis 100 Mol-Äquivalent, vorzugsweise 10 bis 50 Mol-Äquivalent eines tertiären Amins wie 1,4-Diazabicyclo[2,2,2]octan (DABCO) durch.

B: Man setzt ein entsprechendes Sulfonylcarbamat der Formel IV in an sich bekannter Weise (EP-A 120 814) in einem inerten organischen Lösungsmittel bei 0 bis 120 °C, vorzugsweise 10 bis 100 °C mit einem 2-Amino-heteroarylether III um. Man kann hierbei Basen wie tertiäre Amine zusetzen, wodurch die Reaktion beschleunigt und die Produktqualität verbessert wird.
Vorzugsweise arbeitet man bei dieser Variante in einem aprotisch polaren Lösungsmittel wie Dioxan und Tetrahydrofuran in Gegenwart eines tertiären Amins wie p-Dimethylaminopyridin und 1,4-Diazabicyclo[2,2,2]octan.

C: Man setzt ein Sulfonamid der Formel V in an sich bekannter Weise (EP-A 120 814) in einem inerten organischen Lösungsmittel mit ungefähr der stöchiometrischen Menge eines Phenylcarbamats VI bei 0 bis 120 °C, vorzugsweise 25 bis 100 °C um. Man kann auch bei dieser Reaktionsweise Basen wie tertiäre Amine zusetzen. Besonders geeignet sind dabei tertiäre Amine wie Pyridin, die Picoline, 2,4- und 2,6-Lutidin, 2,4,6-Collidin, p-Dimethylaminopyridin, 1,4-Diaza[2,2,2]-bicyclooctan [DABCO] und 1,8-Diazabicyclo[5,4,0]-undec-7-en, vorzugsweise 1,4-Diazabicyclo-[2,2,2]octan und 1,8-Diazabicyclo[5,4,0]undec-7-en.
Vorteilhafterweise arbeitet man auch hier in Lösungsmitteln wie Dioxan und Tetrahydrofuran.

Vorzugsweise nimmt man die Reaktion bei Normaldruck oder unter leichtem Überdruck, etwa bis zu 5 bar, vor, wobei man sowohl diskontinuierlich als auch kontinuierlich arbeiten kann.

Die Salze der Sulfonylharnstoffe erhält man durch Umsetzung mit einer stöchiometrischen Menge eines Metallalkoholats, gegebenenfalls in einem inerten organischen Lösungsmittel.

Die als Ausgangsstoffe der Formel V benötigten Sulfonamide lassen sich aus Halogen-anthranilsäureestern durch Meerwein-Reaktion (F. Muth in "Methoden der Organischen Chemie" (Houben-Weyl) Bd. 9, 557 (1955)) und anschließende Umsetzung der gebildeten Sulfochloride mit Ammoniak herstellen.

Die als Vorprodukte benötigten Aryloxy- bzw. Thioaryl-substituierten Pyrimidine oder Triazine lassen sich nach Literaturmethoden, wie beispielsweise in J. Med. Chem. 29, 676 (1986), in J. Am. Chem. Soc. 73, 2990 (1951), in Arch. Pharm. 296, 151 (1963), in Chem. Ber. 96, 2909 (1963), in Bull. Chem. Soc. Jpn. 45, 3133 (1972), in Agric. Biol. Chem. 30, 896 (1966) und in Rec. Trav. Chim. Pays-Bas 64, 115 (1945) beschrieben, oder in Analogie zu den nachfolgenden Beispielen herstellen.

Im Hinblick auf die biologische Wirksamkeit sind Verbindungen der Formel I bevorzugt, in denen die Substituenten folgende Bedeutung haben:

X Sauerstoff oder Schwefel;

Z Stickstoff oder eine Methingruppe = CH-;

$R^1$ Halogen wie Fluor, Chlor und Brom, insbesondere Chlor, Alkoxycarbonyl wie Methoxycarbonyl, Ethoxycarbonyl, Propyloxycarbonyl, iso-Propyloxycarbonyl und Butoxycarbonyl, insbesondere Methoxycarbonyl und Ethoxycarbonyl,

Alkoxy wie Methoxy, Ethoxy, Propyloxy und 1-Methylethoxy, insbesondere Ethoxy und 1-Methylethoxy, wobei diese Gruppen Halogen wie oben genannt, Alkoxy wie oben genannt sowie Halogenalkoxygruppen wie Trifluormethoxy, Difluormethoxy, Fluormethoxy, Trichlormethoxy, Dichlormethoxy, Chlormethoxy, Difluorchlormethoxy, 1-Fluorethoxy, 2-Fluorethoxy und 2,2,2-Trifluorethoxy, insbesondere aber Trifluormethoxy und Difluormethoxy, Alkylthio wie Methylthio, Ethylthio, Propylthio und 1-Methylethylthio, insbesondere Methyl- und Ethylthio und/oder Haloalkylthio wie Trifluormethylthio, Fluormethylthio, 1-Trifluormethylthio, Fluormethylthio, 1-Fluorethylthio, 2-Fluorethylthio, Chlormethylthio und 2-Chlormethylthio, insbesondere Trifluormethylthio, Chlormethylthio und 2-Chlormethylthio, vorzugsweise in 1 oder 2 Position tragen können, oder entsprechendes Alkoxy sowie Butyloxy, 1-Methylpropyloxy, 2-Methylpropyloxy und 1,1-Dimethylethoxy vorzugsweise in 2 oder 3 Position tragen können;

Carbamoyl wie Carboxamid, N-Methylcarbamoyl, N,N-Dimethylcarbamoyl, N-Ethylcarbamoyl, N,N-Diethylcarbamoyl, N-Ethyl-N-methylcarbamoyl, insbesondere N-Methyl- und N,N-Dimethylcarbamoyl und Hydroxamestergruppen, wie N-Methoxycarbamoyl, N-Methoxy-N-methylcarbamoyl, N-Ethoxycarbamoyl, vorzugsweise N-Methoxy-N-methyl carbamoyl;

$R^2$ Halogen wie unter $R^1$ genannt, vorzugsweise Chlor;

Alkoxy wie unter $R^1$ genannt, vorzugsweise Methoxy;

Alkyl wie Methyl, Ethyl, Propyl, 1-Methylethyl, Butyl, 1-Methylpropyl, 2-Methylpropyl und 1,1-Dimethylpropyl, insbesondere Methyl und Ethyl, wobei diese Gruppen ein- bis dreifach durch Halogenatome und/oder Alkoxygruppen wie unter $R^1$ genannt und/oder durch Alkylthiogruppen wie Methylthio, Ethylthio, Propylthio und 1-Methylethylthio substituiert sein können;

$R^3$ Alkyl wie unter $R^2$ genannt, vorzugsweise Methyl und Ethyl;

Alkoxy wie unter $R^1$ genannt, vorzugsweise Methoxy, Ethoxy und 1-Methylethoxy;

Alkylthio wie unter $R^2$ genannt, insbesondere Methylthio und Ethylthio;

Halogenalkyl wie Trifluormethyl, Difluormethyl, Fluormethyl, Trichlormethyl, Dichlormethyl, Chlormethyl, Difluorchlormethyl, 1-Fluorethyl, 2-Fluorethyl und 2,2,2-Trifluorethyl, insbesondere Trifluormethyl und Difluormethyl;

Halogenalkoxy wie unter $R^1$ genannt, vorzugsweise 2-Chlorethoxy und 2-Fluorethoxy;

Halogenalkylthio wie unter $R^1$ genannt, vorzugsweise 2-Chlorethylthio und 2-Fluorethylthio;

Halogen wie unter $R^1$ genannt, insbesondere Fluor oder Chlor, Cyano, Nitro, Amino und/oder ein- bis zweifach durch die obengenannten Alkylgruppen substituierte Aminogruppen wie Methylamino, Dimethylamino, Ethylamino, Diethylamino, Methylethylamino, Propylamino, N-Methyl-N-propylamino, N-Ethyl-N-propylamino, N,N-Dipropylamino und N,N-Di(1-methylethyl)amino, insbesondere N,N-Dimethylamino;

Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-methyl-3-butenyl, 2-methyl-3-butenyl, 3-Methyl-3-butenyl, 1,1-Dimethyl-2-propenyl, 1-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere Ethenyl, 1-Propenyl, 2-Propenyl und 1-Methylethenyl;

Alkoxycarbonyl, wie im allgemeinen und im besonderen bei $R^1$ genannt; $C_1$-$C_4$-Alkanoyl wie Propionyl und vor allem Acetyl und Pivaloyl oder Benzyl;

ein 5- oder 6-gliedriger über sein Stickstoffatom gebundener gesättigter Heterocyclus, enthaltend neben Methylengruppen ein Stickstoffatom, wobei dieser Cyclus außerdem ein Sauerstoff- oder ein Schwefelatom enthalten kann, wie Pyrrolidinyl, Piperidinyl, Morpholinyl und Thiomorpholinyl;

sowie an benachbarte Ringpositionen gebundenes -O-CRR′-O-, wobei R und R′ Wasserstoff oder Alkyl wie bei $R^3$ genannt, vorzugsweise Wasserstoff, Methyl und Ethyl bedeuten;

Die Anzahl der Substituenten $R^3$ beträgt 0 bis 3, vorzugsweise jedoch 0, 1 oder 2, oder 0 bis 5, wenn $R^3$ Halogen bedeutet, wobei die Gruppen verschieden sein können, wenn m größer oder gleich 2 ist;

$R^4$ Wasserstoff oder Alkyl wie bei $R^2$ genannt, vorzugsweise Wasserstoff und Methyl

$R^5$ die unter $R^3$ genannten Halogenatome, Alkylgruppen, Alkoxygruppen, Alkylthiogruppen und/oder Halogenalkylgruppen.

Die Anzahl der Substituenten $R^5$ beträgt 0 bis 2, wobei die Gruppen verschieden sein können, wenn n 2 bedeutet.

Besonders aktive Verbindungen der Formel I sind in den folgenden Tabellen A, B, C und D aufgeführt.

Tabelle A

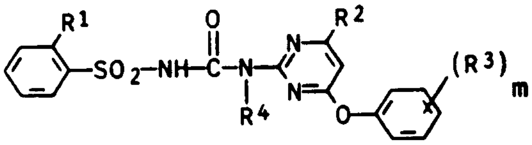

| $R^1$ | $R^4$ | $R^2$ | $(R^3)_m$ |
|---|---|---|---|
| $CO_2CH_3$ | H | Cl | H |
| $CO_2CH_3$ | H | Cl | $3-CH_3$ |
| $CO_2CH_3$ | H | Cl | $3-OCH_3$ |
| $CO_2CH_3$ | H | Cl | $3-CF_3$ |
| $CO_2CH_3$ | H | Cl | 2-F |
| $CO_2CH_3$ | H | Cl | 2-Cl |
| $CO_2CH_3$ | H | Cl | 3-Cl |
| $CO_2CH_3$ | H | Cl | $3-NO_2$ |
| $CO_2CH_3$ | H | Cl | $4-NO_2$ |
| $CO_2CH_3$ | H | Cl | 2-CN |
| $CO_2CH_3$ | H | Cl | $2,6-OCH_3,OCH_3$ |
| $CO_2CH_3$ | H | Cl | 2,4,6-Cl,Cl,Cl |
| $CO_2CH_3$ | H | Cl | $2-Cl,4-CF_3$ |
| $CO_2CH_3$ | H | Cl | $3-N(CH_3)_2$ |
| $CO_2CH_3$ | $CH_3$ | Cl | H |
| $CO_2CH_3$ | $CH_3$ | Cl | $3-CH_3$ |
| $CO_2CH_3$ | $CH_3$ | Cl | $3-OCH_3$ |
| $CO_2CH_3$ | $CH_3$ | Cl | $3-CF_3$ |
| $CO_2CH_3$ | $CH_3$ | Cl | 2-F |
| $CO_2CH_3$ | $CH_3$ | Cl | 2-Cl |
| $CO_2CH_3$ | $CH_3$ | Cl | 3-Cl |
| $CO_2CH_3$ | $CH_3$ | Cl | $3-NO_2$ |
| $CO_2CH_3$ | $CH_3$ | Cl | $4-NO_2$ |
| $CO_2CH_3$ | $CH_3$ | Cl | 2-CN |
| $CO_2CH_3$ | $CH_3$ | Cl | $2,6-OCH_3,OCH_3$ |
| $CO_2CH_3$ | $CH_3$ | Cl | 2,4,6-Cl,Cl,Cl |
| $CO_2CH_3$ | $CH_3$ | Cl | $2-Cl,4-CF_3$ |
| $CO_2CH_3$ | $CH_3$ | Cl | $3-N(CH_3)_2$ |

Fortsetzung Tabelle A

| $R^1$ | $R^4$ | $R^2$ | $(R^3)_m$ |
|---|---|---|---|
| $CO_2CH_3$ | H | $CH_3$ | H |
| $CO_2CH_3$ | H | $CH_3$ | $3-CH_3$ |
| $CO_2CH_3$ | H | $CH_3$ | $3-OCH_3$ |
| $CO_2CH_3$ | H | $CH_3$ | $3-CF_3$ |
| $CO_2CH_3$ | H | $CH_3$ | 2-F |
| $CO_2CH_3$ | H | $CH_3$ | 2-Cl |
| $CO_2CH_3$ | H | $CH_3$ | 3-Cl |
| $CO_2CH_3$ | H | $CH_3$ | $3-NO_2$ |
| $CO_2CH_3$ | H | $CH_3$ | $4-NO_2$ |
| $CO_2CH_3$ | H | $CH_3$ | 2-CN |
| $CO_2CH_3$ | H | $CH_3$ | $2,6-OCH_3,OCH_3$ |
| $CO_2CH_3$ | H | $CH_3$ | 2,4,6-Cl,Cl,Cl |
| $CO_2CH_3$ | H | $CH_3$ | $2-Cl,4-CF_3$ |
| $CO_2CH_3$ | H | $CH_3$ | $3-N(CH_3)_2$ |
| $CO_2CH_3$ | H | $CF_3$ | H |
| $CO_2CH_3$ | H | $CF_3$ | $3-CH_3$ |
| $CO_2CH_3$ | H | $CF_3$ | $3-OCH_3$ |
| $CO_2CH_3$ | H | $CF_3$ | $3-CF_3$ |
| $CO_2CH_3$ | H | $CF_3$ | 2-F |
| $CO_2CH_3$ | H | $CF_3$ | 2-Cl |
| $CO_2CH_3$ | H | $CF_3$ | 3-Cl |
| $CO_2CH_3$ | H | $CF_3$ | $3-NO_2$ |
| $CO_2CH_3$ | H | $CF_3$ | $4-NO_2$ |
| $CO_2CH_3$ | H | $CF_3$ | 2-CN |
| $CO_2CH_3$ | H | $CF_3$ | $2,6-OCH_3,OCH_3$ |
| $CO_2CH_3$ | H | $CF_3$ | 2,4,6-Cl,Cl,Cl |
| $CO_2CH_3$ | H | $CF_3$ | $2-Cl,4-CF_3$ |
| $CO_2CH_3$ | H | $CF_3$ | $3-N(CH_3)_2$ |
| $CO_2CH_3$ | H | $OCH_3$ | $2-OCH_3$ |
| $CO_2CH_3$ | H | $OCH_3$ | $4-OCH_3$ |
| $CO_2CH_3$ | H | $OCH_3$ | $2-OCH_2CH_3$ |
| $CO_2CH_3$ | H | $OCH_3$ | $3-OCH_2CH_3$ |
| $CO_2CH_3$ | H | $OCH_3$ | $4-OCH_2CH_3$ |
| $CO_2CH_3$ | H | $OCH_3$ | $2-OCH(CH_3)_2$ |
| $CO_2CH_3$ | H | $OCH_3$ | $3-OCH(CH_3)_2$ |
| $CO_2CH_3$ | H | $OCH_3$ | $4-OCH(CH_3)_2$ |
| $CO_2CH_3$ | H | $OCH_3$ | $2-O(CH_2)_2CH_3$ |
| $CO_2CH_3$ | H | $OCH_3$ | $3-O(CH_2)_2CH_3$ |
| $CO_2CH_3$ | H | $OCH_3$ | $4-O(CH_2)_2CH_3$ |

Fortsetzung Tabelle A

| $R^1$ | $R^4$ | $R^2$ | $(R^3)_m$ |
|---|---|---|---|
| $CO_2CH_3$ | H | $OCH_3$ | $2-OC(CH_3)_3$ |
| $CO_2CH_3$ | H | $OCH_3$ | $3-OC(CH_3)_3$ |
| $CO_2CH_3$ | H | $OCH_3$ | $4-OC(CH_3)_3$ |
| $CO_2CH_3$ | H | $OCH_3$ | $2,3-OCH_3,OCH_3$ |
| $CO_2CH_3$ | H | $OCH_3$ | $2,4-OCH_3,OCH_3$ |
| $CO_2CH_3$ | H | $OCH_3$ | $2,5-OCH_3,OCH_3$ |
| $CO_2CH_3$ | H | $OCH_3$ | $3,4-OCH_3,OCH_3$ |
| $CO_2CH_3$ | H | $OCH_3$ | $3,5-OCH_3,OCH_3$ |
| $CO_2CH_3$ | H | $OCH_3$ | $2,3,4-OCH_3,OCH_3,OCH_3$ |
| $CO_2CH_3$ | H | $OCH_3$ | $2,3,5-OCH_3,OCH_3,OCH_3$ |
| $CO_2CH_3$ | H | $OCH_3$ | $2,4,6-OCH_3,OCH_3,OCH_3$ |
| $CO_2CH_3$ | H | $OCH_3$ | $3,4,5-OCH_3,OCH_3,OCH_3$ |
| $CO_2CH_3$ | H | $OCH_3$ | H |
| $CO_2CH_3$ | H | $OCH_3$ | $3-CH_3$ |
| $CO_2CH_3$ | H | $OCH_3$ | $3-OCH_3$ |
| $CO_2CH_3$ | H | $OCH_3$ | $3-CF_3$ |
| $CO_2CH_3$ | H | $OCH_3$ | 2-F |
| $CO_2CH_3$ | H | $OCH_3$ | 2-Cl |
| $CO_2CH_3$ | H | $OCH_3$ | 3-Cl |
| $CO_2CH_3$ | H | $OCH_3$ | $3-NO_2$ |
| $CO_2CH_3$ | H | $OCH_3$ | $4-NO_2$ |
| $CO_2CH_3$ | H | $OCH_3$ | 2-CN |
| $CO_2CH_3$ | H | $OCH_3$ | $2,6-OCH_3,OCH_3$ |
| $CO_2CH_3$ | H | $OCH_3$ | 2,4,6-Cl,Cl,Cl |
| $CO_2CH_3$ | H | $OCH_3$ | $2-Cl,4-CF_3$ |
| $CO_2CH_3$ | H | $OCH_3$ | $3-N(CH_3)_2$ |
| Cl | H | Cl | H |
| Cl | H | Cl | $3-CH_3$ |
| Cl | H | Cl | $3-OCH_3$ |
| Cl | H | Cl | $3-CF_3$ |
| Cl | H | Cl | 2-F |
| Cl | H | Cl | 2-Cl |
| Cl | H | Cl | 3-Cl |
| Cl | H | Cl | $3-NO_2$ |
| Cl | H | Cl | $4-NO_2$ |
| Cl | H | Cl | 2-CN |
| Cl | H | Cl | $2,6-OCH_3,OCH_3$ |
| Cl | H | Cl | 2,4,6-Cl,Cl,Cl |
| Cl | H | Cl | $2-Cl,4-CF_3$ |
| Cl | H | Cl | $3-N(CH_3)_2$ |

Fortsetzung Tabelle A

| $R^1$ | $R^4$ | $R^2$ | $(R^3)_m$ |
|---|---|---|---|
| Cl | $CH_3$ | Cl | H |
| Cl | $CH_3$ | Cl | 3-$CH_3$ |
| Cl | $CH_3$ | Cl | 3-$OCH_3$ |
| Cl | $CH_3$ | Cl | 3-$CF_3$ |
| Cl | $CH_3$ | Cl | 2-F |
| Cl | $CH_3$ | Cl | 2-Cl |
| Cl | $CH_3$ | Cl | 3-Cl |
| Cl | $CH_3$ | Cl | 3-$NO_2$ |
| Cl | $CH_3$ | Cl | 4-$NO_2$ |
| Cl | $CH_3$ | Cl | 2-CN |
| Cl | $CH_3$ | Cl | 2,6-$OCH_3$,$OCH_3$ |
| Cl | $CH_3$ | Cl | 2,4,6-Cl,Cl,Cl |
| Cl | $CH_3$ | Cl | 2-Cl,4-$CF_3$ |
| Cl | $CH_3$ | Cl | 3-$N(CH_3)_2$ |
| Cl | H | $CH_3$ | H |
| Cl | H | $CH_3$ | 3-$CH_3$ |
| Cl | H | $CH_3$ | 3-$OCH_3$ |
| Cl | H | $CH_3$ | 3-$CF_3$ |
| Cl | H | $CH_3$ | 2-F |
| Cl | H | $CH_3$ | 2-Cl |
| Cl | H | $CH_3$ | 3-Cl |
| Cl | H | $CH_3$ | 3-$NO_2$ |
| Cl | H | $CH_3$ | 4-$NO_2$ |
| Cl | H | $CH_3$ | 2-CN |
| Cl | H | $CH_3$ | 2,6-$OCH_3$,$OCH_3$ |
| Cl | H | $CH_3$ | 2,4,6-Cl,Cl,Cl |
| Cl | H | $CH_3$ | 2-Cl,4-$CF_3$ |
| Cl | H | $CH_3$ | 3-$N(CH_3)_2$ |
| Cl | H | $CF_3$ | H |
| Cl | H | $CF_3$ | 3-$CH_3$ |
| Cl | H | $CF_3$ | 3-$OCH_3$ |
| Cl | H | $CF_3$ | 3-$CF_3$ |
| Cl | H | $CF_3$ | 2-F |
| Cl | H | $CF_3$ | 2-Cl |
| Cl | H | $CF_3$ | 3-Cl |

Fortsetzung Tabelle A

| $R^1$ | $R^4$ | $R^2$ | $(R^3)_m$ |
|---|---|---|---|
| Cl | H | $CF_3$ | 3-$NO_2$ |
| Cl | H | $CF_3$ | 4-$NO_2$ |
| Cl | H | $CF_3$ | 2-CN |
| Cl | H | $CF_3$ | 2,6-$OCH_3$,$OCH_3$ |
| Cl | H | $CF_3$ | 2,4,6-Cl,Cl,Cl |
| Cl | H | $CF_3$ | 2-Cl,4-$CF_3$ |
| Cl | H | $CF_3$ | 3-$N(CH_3)_2$ |
| Cl | H | $OCH_3$ | H |
| Cl | H | $OCH_3$ | 3-$CH_3$ |
| Cl | H | $OCH_3$ | 3-$OCH_3$ |
| Cl | H | $OCH_3$ | 3-$CF_3$ |
| Cl | H | $OCH_3$ | 2-F |
| Cl | H | $OCH_3$ | 2-Cl |
| Cl | H | $OCH_3$ | 3-Cl |
| Cl | H | $OCH_3$ | 3-$NO_2$ |
| Cl | H | $OCH_3$ | 4-$NO_2$ |
| Cl | H | $OCH_3$ | 2-CN |
| Cl | H | $OCH_3$ | 2,6-$OCH_3$,$OCH_3$ |
| Cl | H | $OCH_3$ | 2,4,6-Cl,Cl,Cl |
| Cl | H | $OCH_3$ | 2-Cl,4-$CF_3$ |
| Cl | H | $OCH_3$ | 3-$N(CH_3)_2$ |
| Cl | H | $OCH_3$ | 2-$OCH_3$ |
| Cl | H | $OCH_3$ | 4-$OCH_3$ |
| Cl | H | $OCH_3$ | 2-$OCH_2CH_3$ |
| Cl | H | $OCH_3$ | 3-$OCH_2CH_3$ |
| Cl | H | $OCH_3$ | 4-$OCH_2CH_3$ |
| Cl | H | $OCH_3$ | 2-$OCH(CH_3)_2$ |
| Cl | H | $OCH_3$ | 3-$OCH(CH_3)_2$ |
| Cl | H | $OCH_3$ | 4-$OCH(CH_3)_2$ |
| Cl | H | $OCH_3$ | 2-$O(CH_2)_2CH_3$ |
| Cl | H | $OCH_3$ | 3-$O(CH_2)_2CH_3$ |
| Cl | H | $OCH_3$ | 4-$O(CH_2)_2CH_3$ |
| Cl | H | $OCH_3$ | 2-$OC(CH_3)_3$ |
| Cl | H | $OCH_3$ | 3-$OC(CH_3)_3$ |
| Cl | H | $OCH_3$ | 4-$OC(CH_3)_3$ |
| Cl | H | $OCH_3$ | 2,3-$OCH_3$,$OCH_3$ |
| Cl | H | $OCH_3$ | 2,4-$OCH_3$,$OCH_3$ |
| Cl | H | $OCH_3$ | 2,5-$OCH_3$,$OCH_3$ |
| Cl | H | $OCH_3$ | 3,4-$OCH_3$,$OCH_3$ |
| Cl | H | $OCH_3$ | 3,5-$OCH_3$,$OCH_3$ |

Fortsetzung Tabelle A

| $R^1$ | $R^4$ | $R^2$ | $(R^3)_m$ |
|---|---|---|---|
| Cl | H | $OCH_3$ | 2,3,4-$OCH_3$,$OCH_3$,$OCH_3$ |
| Cl | H | $OCH_3$ | 2,3,5-$OCH_3$,$OCH_3$,$OCH_3$ |
| Cl | H | $OCH_3$ | 2,4,6-$OCH_3$,$OCH_3$,$OCH_3$ |
| Cl | H | $OCH_3$ | 3,4,5-$OCH_3$,$OCH_3$,$OCH_3$ |
| $CO_2C_2H_5$ | H | Cl | H |
| $CO_2C_2H_5$ | H | $CH_3$ | 3-$SCH_3$ |
| $CO_2C_2H_5$ | $CH_3$ | $CH_3$ | 2-$O(CH_2)_2Cl$ |
| $CO_2C_2H_5$ | H | Cl | 3-$OCCl_3$ |
| $CO_2C_2H_5$ | H | $OCH_3$ | 4-$OCH_2F$ |
| $CO_2C_2H_5$ | H | $OCH_3$ | 4-$OCHF_2$ |
| $CO_2C_3H_7$ | H | $CF_3$ | 3-$O(CH_2)_2F$ |
| $CO_2C_3H_7$ | H | $CF_3$ | 2-$S(CH_2)_2Cl$ |
| $CO_2C_3H_7$ | H | $CCl_3$ | 2-$S(CH_2)_2F$ |
| $CO_2C_3H_7$ | H | $CH_3$ | 2-$SCF_3$ |
| $CO_2C_3H_7$ | $CH_3$ | Cl | 4-$SCHF_2$ |
| $CONH(CH_3)$ | H | Cl | 2-$OCH_3$ |
| $CONH(CH_3)$ | H | $CH_3$ | 3-$OCH_3$ |
| $CON(CH_3)_2$ | H | Cl | 2-$OCH_3$ |
| $CON(CH_3)_2$ | H | $CH_3$ | 3-$OCH_3$ |
| $CON(CH_3)_2$ | H | $OCH_3$ | 4-$OCH_3$ |
| $CON(CH_3)_2$ | H | $CF_3$ | 2-$OCH_3$ |
| $CON(CH_3)_2$ | H | $OC_2H_5$ | 2-$SCH_3$ |
| $CON(CH_3)_2$ | H | $OC_2H_5$ | 3-$OCF_3$ |
| $CON(CH_3)OCH_3$ | H | $CH_3$ | 2-$OCH_3$ |
| $CON(CH_3)OCH_3$ | H | $OCH_3$ | 3-$OCH_3$ |
| $CON(CH_3)OCH_3$ | H | Cl | 2-$OCH_3$ |
| $CO_2(CH_2)_2Cl$ | H | Cl | 2-$N(CH_3)_2$ |
| $CO_2(CH_2)_2OCH_3$ | H | Cl | 2-CN |
| $CO_2(CH_2)_2OC_2H_5$ | $CH_3$ | Cl | 2-$NO_2$ |
| $CO_2(CH_2)_2OCF_3$ | H | Cl | 2,3-Cl,Cl |
| $OCH_3$ | H | Cl | 2-Cl |
| $OCH_3$ | H | Cl | 3-$NO_2$ |
| $OC_2H_5$ | H | Cl | 4-CN |
| $O(CH_2)_2Cl$ | H | $CH_3$ | 3-$CH_3$ |
| $O(CH_2)_2F$ | $CH_3$ | $CH_3$ | 2-Cl,4-$CF_3$ |
| $O(CH_2)_2OCH_3$ | $CH_3$ | $CH_3$ | 2,3,5-Cl,Cl,Cl |
| $O(CH_2)_2OCH_3$ | $CH_3$ | $CH_3$ | 3-$N(CH_3)_2$ |
| $O(CH_2)_2SCH_3$ | $CH_3$ | $CH_3$ | 4-$OCH_3$ |
| $O(CH_2)_2SCH_3$ | $CH_3$ | $OCH_3$ | 2,4-Cl,Cl |
| $O(CH_2)_2SCF_3$ | $CH_3$ | $OCH_3$ | 2-$OCH_3$ |

Tabelle B

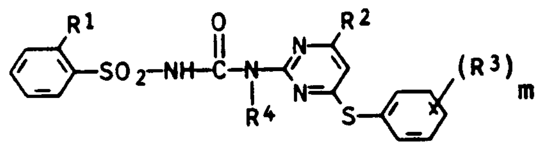

| $R^1$ | $R^4$ | $R^2$ | $(R^3)_m$ |
|---|---|---|---|
| $CO_2CH_3$ | H | Cl | H |
| $CO_2CH_3$ | H | Cl | 3-$CH_3$ |
| $CO_2CH_3$ | H | Cl | 3-$OCH_3$ |
| $CO_2CH_3$ | H | Cl | 3-$CF_3$ |
| $CO_2CH_3$ | H | Cl | 2-F |
| $CO_2CH_3$ | H | Cl | 2-Cl |
| $CO_2CH_3$ | H | Cl | 3-Cl |
| $CO_2CH_3$ | H | Cl | 3-$NO_2$ |
| $CO_2CH_3$ | H | Cl | 4-$NO_2$ |
| $CO_2CH_3$ | H | Cl | 2-CN |
| $CO_2CH_3$ | H | Cl | 2,6-$OCH_3$,$OCH_3$ |
| $CO_2CH_3$ | H | Cl | 2,4,6-Cl,Cl,Cl |
| $CO_2CH_3$ | H | Cl | 2-Cl,4-$CF_3$ |
| $CO_2CH_3$ | H | Cl | 3-$N(CH_3)_2$ |
| $CO_2CH_3$ | $CH_3$ | Cl | H |
| $CO_2CH_3$ | $CH_3$ | Cl | 3-$CH_3$ |
| $CO_2CH_3$ | $CH_3$ | Cl | 3-$OCH_3$ |
| $CO_2CH_3$ | $CH_3$ | Cl | 3-$CF_3$ |
| $CO_2CH_3$ | $CH_3$ | Cl | 2-F |
| $CO_2CH_3$ | $CH_3$ | Cl | 2-Cl |
| $CO_2CH_3$ | $CH_3$ | Cl | 3-Cl |
| $CO_2CH_3$ | $CH_3$ | Cl | 3-$NO_2$ |
| $CO_2CH_3$ | $CH_3$ | Cl | 4-$NO_2$ |
| $CO_2CH_3$ | $CH_3$ | Cl | 2-CN |
| $CO_2CH_3$ | $CH_3$ | Cl | 2,6-$OCH_3$,$OCH_3$ |
| $CO_2CH_3$ | $CH_3$ | Cl | 2,4,6-Cl,Cl,Cl |
| $CO_2CH_3$ | $CH_3$ | Cl | 2-Cl,4-$CF_3$ |
| $CO_2CH_3$ | $CH_3$ | Cl | 3-$N(CH_3)_2$ |

Fortsetzung Tabelle B

| $R^1$ | $R^4$ | $R^2$ | $(R^3)_m$ |
|---|---|---|---|
| $CO_2CH_3$ | H | $CH_3$ | H |
| $CO_2CH_3$ | H | $CH_3$ | 3-$CH_3$ |
| $CO_2CH_3$ | H | $CH_3$ | 3-$OCH_3$ |
| $CO_2CH_3$ | H | $CH_3$ | 3-$CF_3$ |
| $CO_2CH_3$ | H | $CH_3$ | 2-F |
| $CO_2CH_3$ | H | $CH_3$ | 2-Cl |
| $CO_2CH_3$ | H | $CH_3$ | 3-Cl |
| $CO_2CH_3$ | H | $CH_3$ | 3-$NO_2$ |
| $CO_2CH_3$ | H | $CH_3$ | 4-$NO_2$ |
| $CO_2CH_3$ | H | $CH_3$ | 2-CN |
| $CO_2CH_3$ | H | $CH_3$ | 2,6-$OCH_3$,$OCH_3$ |
| $CO_2CH_3$ | H | $CH_3$ | 2,4,6-Cl,Cl,Cl |
| $CO_2CH_3$ | H | $CH_3$ | 2-Cl,4-$CF_3$ |
| $CO_2CH_3$ | H | $CH_3$ | 3-$N(CH_3)_2$ |
| $CO_2CH_3$ | H | $CF_3$ | H |
| $CO_2CH_3$ | H | $CF_3$ | 3-$CH_3$ |
| $CO_2CH_3$ | H | $CF_3$ | 3-$OCH_3$ |
| $CO_2CH_3$ | H | $CF_3$ | 3-$CF_3$ |
| $CO_2CH_3$ | H | $CF_3$ | 2-F |
| $CO_2CH_3$ | H | $CF_3$ | 2-Cl |
| $CO_2CH_3$ | H | $CF_3$ | 3-Cl |
| $CO_2CH_3$ | H | $CF_3$ | 3-$NO_2$ |
| $CO_2CH_3$ | H | $CF_3$ | 4-$NO_2$ |
| $CO_2CH_3$ | H | $CF_3$ | 2-CN |
| $CO_2CH_3$ | H | $CF_3$ | 2,6-$OCH_3$,$OCH_3$ |
| $CO_2CH_3$ | H | $CF_3$ | 2,4,6-Cl,Cl,Cl |
| $CO_2CH_3$ | H | $CF_3$ | 2-Cl,4-$CF_3$ |
| $CO_2CH_3$ | H | $CF_3$ | 3-$N(CH_3)_2$ |

Fortsetzung Tabelle B

| $R^1$ | $R^4$ | $R^2$ | $(R^3)_m$ |
|---|---|---|---|
| $CO_2CH_3$ | H | $OCH_3$ | H |
| $CO_2CH_3$ | H | $OCH_3$ | 3-$CH_3$ |
| $CO_2CH_3$ | H | $OCH_3$ | 3-$OCH_3$ |
| $CO_2CH_3$ | H | $OCH_3$ | 3-$CF_3$ |
| $CO_2CH_3$ | H | $OCH_3$ | 2-F |
| $CO_2CH_3$ | H | $OCH_3$ | 2-Cl |
| $CO_2CH_3$ | H | $OCH_3$ | 3-Cl |
| $CO_2CH_3$ | H | $OCH_3$ | 3-$NO_2$ |
| $CO_2CH_3$ | H | $OCH_3$ | 4-$NO_2$ |
| $CO_2CH_3$ | H | $OCH_3$ | 2-CN |
| $CO_2CH_3$ | H | $OCH_3$ | 2,6-$OCH_3$,$OCH_3$ |
| $CO_2CH_3$ | H | $OCH_3$ | 2,4,6-Cl,Cl,Cl |
| $CO_2CH_3$ | H | $OCH_3$ | 2-Cl,4-$CF_3$ |
| $CO_2CH_3$ | H | $OCH_3$ | 3-$N(CH_3)_2$ |
| Cl | H | Cl | H |
| Cl | H | Cl | 3-$CH_3$ |
| Cl | H | Cl | 3-$OCH_3$ |
| Cl | H | Cl | 3-$CF_3$ |
| Cl | H | Cl | 2-F |
| Cl | H | Cl | 2-Cl |
| Cl | H | Cl | 3-Cl |
| Cl | H | Cl | 3-$NO_2$ |
| Cl | H | Cl | 4-$NO_2$ |
| Cl | H | Cl | 2-CN |
| Cl | H | Cl | 2,6-$OCH_3$,$OCH_3$ |
| Cl | H | Cl | 2,4,6-Cl,Cl,Cl |
| Cl | H | Cl | 2-Cl,4-$CF_3$ |
| Cl | H | Cl | 3-$N(CH_3)_2$ |

Fortsetzung Tabelle B

| $R^1$ | $R^4$ | $R^2$ | $(R^3)_m$ |
|---|---|---|---|
| Cl | $CH_3$ | Cl | H |
| Cl | $CH_3$ | Cl | 3-$CH_3$ |
| Cl | $CH_3$ | Cl | 3-$OCH_3$ |
| Cl | $CH_3$ | Cl | 3-$CF_3$ |
| Cl | $CH_3$ | Cl | 2-F |
| Cl | $CH_3$ | Cl | 2-Cl |
| Cl | $CH_3$ | Cl | 3-Cl |
| Cl | $CH_3$ | Cl | 3-$NO_2$ |
| Cl | $CH_3$ | Cl | 4-$NO_2$ |
| Cl | $CH_3$ | Cl | 2-CN |
| Cl | $CH_3$ | Cl | 2,6-$OCH_3$,$OCH_3$ |
| Cl | $CH_3$ | Cl | 2,4,6-Cl,Cl,Cl |
| Cl | $CH_3$ | Cl | 2-Cl,4-$CF_3$ |
| Cl | $CH_3$ | Cl | 3-$N(CH_3)_2$ |
| Cl | H | $CH_3$ | H |
| Cl | H | $CH_3$ | 3-$CH_3$ |
| Cl | H | $CH_3$ | 3-$OCH_3$ |
| Cl | H | $CH_3$ | 3-$CF_3$ |
| Cl | H | $CH_3$ | 2-F |
| Cl | H | $CH_3$ | 2-Cl |
| Cl | H | $CH_3$ | 3-Cl |
| Cl | H | $CH_3$ | 3-$NO_2$ |
| Cl | H | $CH_3$ | 4-$NO_2$ |
| Cl | H | $CH_3$ | 2-CN |
| Cl | H | $CH_3$ | 2,6-$OCH_3$,$OCH_3$ |
| Cl | H | $CH_3$ | 2,4,6-Cl,Cl,Cl |
| Cl | H | $CH_3$ | 2-Cl,4-$CF_3$ |
| Cl | H | $CH_3$ | 3-$N(CH_3)_2$ |

Fortsetzung Tabelle B

| $R^1$ | $R^4$ | $R^2$ | $(R^3)_m$ |
|---|---|---|---|
| Cl | H | $CF_3$ | H |
| Cl | H | $CF_3$ | 3-$CH_3$ |
| Cl | H | $CF_3$ | 3-$OCH_3$ |
| Cl | H | $CF_3$ | 3-$CF_3$ |
| Cl | H | $CF_3$ | 2-F |
| Cl | H | $CF_3$ | 2-Cl |
| Cl | H | $CF_3$ | 3-Cl |
| Cl | H | $CF_3$ | 3-$NO_2$ |
| Cl | H | $CF_3$ | 4-$NO_2$ |
| Cl | H | $CF_3$ | 2-CN |
| Cl | H | $CF_3$ | 2,6-$OCH_3$,$OCH_3$ |
| Cl | H | $CF_3$ | 2,4,6-Cl,Cl,Cl |
| Cl | H | $CF_3$ | 2-Cl,4-$CF_3$ |
| Cl | H | $CF_3$ | 3-$N(CH_3)_2$ |
| Cl | H | $OCH_3$ | H |
| Cl | H | $OCH_3$ | 3-$CH_3$ |
| Cl | H | $OCH_3$ | 3-$OCH_3$ |
| Cl | H | $OCH_3$ | 3-$CF_3$ |
| Cl | H | $OCH_3$ | 2-F |
| Cl | H | $OCH_3$ | 2-Cl |
| Cl | H | $OCH_3$ | 3-Cl |
| Cl | H | $OCH_3$ | 3-$NO_2$ |
| Cl | H | $OCH_3$ | 4-$NO_2$ |
| Cl | H | $OCH_3$ | 2-CN |
| Cl | H | $OCH_3$ | 2,6-$OCH_3$,$OCH_3$ |
| Cl | H | $OCH_3$ | 2,4,6-Cl,Cl,Cl |
| Cl | H | $OCH_3$ | 2-Cl,4-$CF_3$ |
| Cl | H | $OCH_3$ | 3-$N(CH_3)_2$ |

Fortsetzung Tabelle B

| $R^1$ | $R^4$ | $R^2$ | $(R^3)_m$ |
|---|---|---|---|
| $CO_2C_2H_5$ | H | Cl | H |
| $CO_2C_2H_5$ | H | $CH_3$ | 3-$SCH_3$ |
| $CO_2C_2H_5$ | $CH_3$ | $CH_3$ | 2-$O(CH_2)_2Cl$ |
| $CO_2C_2H_5$ | H | Cl | 3-$OCCl_3$ |
| $CO_2C_2H_5$ | H | $OCH_3$ | 4-$OCH_2F$ |
| $CO_2C_2H_5$ | H | $OCH_3$ | 4-$OCHF_2$ |
| $CO_2C_3H_7$ | H | $CF_3$ | 3-$O(CH_2)_2F$ |
| $CO_2C_3H_7$ | H | $CF_3$ | 2-$S(CH_2)_2Cl$ |
| $CO_2C_3H_7$ | H | $CCl_3$ | 2-$S(CH_2)_2F$ |
| $CO_2C_3H_7$ | H | $CH_3$ | 2-$SCF_3$ |
| $CO_2C_3H_7$ | $CH_3$ | Cl | 4-$SCHF_2$ |
| $CONH(CH_3)$ | H | Cl | 2-$OCH_3$ |
| $CONH(CH_3)$ | H | $CH_3$ | 3-$OCH_3$ |
| $CON(CH_3)_2$ | H | Cl | 2-$OCH_3$ |
| $CON(CH_3)_2$ | H | $CH_3$ | 3-$OCH_3$ |
| $CON(CH_3)_2$ | H | $OCH_3$ | 4-$OCH_3$ |
| $CON(CH_3)_2$ | H | $CF_3$ | 2-$OCH_3$ |
| $CON(CH_3)_2$ | H | $OC_2H_5$ | 2-$SCH_3$ |
| $CON(CH_3)_2$ | H | $OC_2H_5$ | 3-$OCF_3$ |
| $CON(CH_3)OCH_3$ | H | $CH_3$ | 2-$OCH_3$ |
| $CON(CH_3)OCH_3$ | H | $OCH_3$ | 3-$OCH_3$ |
| $CON(CH_3)OCH_3$ | H | Cl | 2-$OCH_3$ |
| $CO_2(CH_2)_2Cl$ | H | Cl | 2-$N(CH_3)_2$ |
| $CO_2(CH_2)_2OCH_3$ | H | Cl | 2-CN |
| $CO_2(CH_2)_2OC_2H_5$ | $CH_3$ | Cl | 2-$NO_2$ |
| $CO_2(CH_2)_2OCF_3$ | H | Cl | 2,3-Cl,Cl |
| $OCH_3$ | H | Cl | 2-Cl |
| $OCH_3$ | H | Cl | 3-$NO_2$ |
| $OC_2H_5$ | H | Cl | 4-CN |
| $O(CH_2)_2Cl$ | H | $CH_3$ | 3-$CH_3$ |
| $O(CH_2)_2F$ | $CH_3$ | $CH_3$ | 2-Cl,4-$CF_3$ |
| $O(CH_2)_2OCH_3$ | $CH_3$ | $CH_3$ | 2,3,5-Cl,Cl,Cl |
| $O(CH_2)_2OCH_3$ | $CH_3$ | $CH_3$ | 3-$N(CH_3)_2$ |
| $O(CH_2)_2SCH_3$ | $CH_3$ | $CH_3$ | 4-$OCH_3$ |
| $O(CH_2)_2SCH_3$ | $CH_3$ | $OCH_3$ | 2,4-Cl,Cl |
| $O(CH_2)_2SCF_3$ | $CH_3$ | $OCH_3$ | 2-$OCH_3$ |

Tabelle C

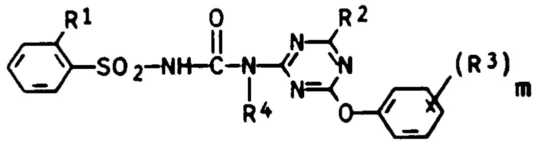

| $R^1$ | $R^4$ | $R^2$ | $(R^3)_m$ |
|---|---|---|---|
| $CO_2CH_3$ | H | Cl | H |
| $CO_2CH_3$ | H | Cl | 3-$CH_3$ |
| $CO_2CH_3$ | H | Cl | 3-$OCH_3$ |
| $CO_2CH_3$ | H | Cl | 3-$CF_3$ |
| $CO_2CH_3$ | H | Cl | 2-F |
| $CO_2CH_3$ | H | Cl | 2-Cl |
| $CO_2CH_3$ | H | Cl | 3-Cl |
| $CO_2CH_3$ | H | Cl | 3-$NO_2$ |
| $CO_2CH_3$ | H | Cl | 4-$NO_2$ |
| $CO_2CH_3$ | H | Cl | 2-CN |
| $CO_2CH_3$ | H | Cl | 2,6-$OCH_3$,$OCH_3$ |
| $CO_2CH_3$ | H | Cl | 2,4,6-Cl,Cl,Cl |
| $CO_2CH_3$ | H | Cl | 2-Cl,4-$CF_3$ |
| $CO_2CH_3$ | H | Cl | 3-$N(CH_3)_2$ |
| $CO_2CH_3$ | $CH_3$ | Cl | H |
| $CO_2CH_3$ | $CH_3$ | Cl | 3-$CH_3$ |
| $CO_2CH_3$ | $CH_3$ | Cl | 3-$OCH_3$ |
| $CO_2CH_3$ | $CH_3$ | Cl | 3-$CF_3$ |
| $CO_2CH_3$ | $CH_3$ | Cl | 2-F |
| $CO_2CH_3$ | $CH_3$ | Cl | 2-Cl |
| $CO_2CH_3$ | $CH_3$ | Cl | 3-Cl |
| $CO_2CH_3$ | $CH_3$ | Cl | 3-$NO_2$ |
| $CO_2CH_3$ | $CH_3$ | Cl | 4-$NO_2$ |
| $CO_2CH_3$ | $CH_3$ | Cl | 2-CN |
| $CO_2CH_3$ | $CH_3$ | Cl | 2,6-$OCH_3$,$OCH_3$ |
| $CO_2CH_3$ | $CH_3$ | Cl | 2,4,6-Cl,Cl,Cl |
| $CO_2CH_3$ | $CH_3$ | Cl | 2-Cl,4-$CF_3$ |
| $CO_2CH_3$ | $CH_3$ | Cl | 3-$N(CH_3)_2$ |

Fortsetzung Tabelle C

| $R^1$ | $R^4$ | $R^2$ | $(R^3)_m$ |
|---|---|---|---|
| $CO_2CH_3$ | H | $CH_3$ | H |
| $CO_2CH_3$ | H | $CH_3$ | 3-$CH_3$ |
| $CO_2CH_3$ | H | $CH_3$ | 3-$OCH_3$ |
| $CO_2CH_3$ | H | $CH_3$ | 3-$CF_3$ |
| $CO_2CH_3$ | H | $CH_3$ | 2-F |
| $CO_2CH_3$ | H | $CH_3$ | 2-Cl |
| $CO_2CH_3$ | H | $CH_3$ | 3-Cl |
| $CO_2CH_3$ | H | $CH_3$ | 3-$NO_2$ |
| $CO_2CH_3$ | H | $CH_3$ | 4-$NO_2$ |
| $CO_2CH_3$ | H | $CH_3$ | 2-CN |
| $CO_2CH_3$ | H | $CH_3$ | 2,6-$OCH_3$,$OCH_3$ |
| $CO_2CH_3$ | H | $CH_3$ | 2,4,6-Cl,Cl,Cl |
| $CO_2CH_3$ | H | $CH_3$ | 2-Cl,4-$CF_3$ |
| $CO_2CH_3$ | H | $CH_3$ | 3-$N(CH_3)_2$ |
| $CO_2CH_3$ | H | $CF_3$ | H |
| $CO_2CH_3$ | H | $CF_3$ | 3-$CH_3$ |
| $CO_2CH_3$ | H | $CF_3$ | 3-$OCH_3$ |
| $CO_2CH_3$ | H | $CF_3$ | 3-$CF_3$ |
| $CO_2CH_3$ | H | $CF_3$ | 2-F |
| $CO_2CH_3$ | H | $CF_3$ | 2-Cl |
| $CO_2CH_3$ | H | $CF_3$ | 3-Cl |
| $CO_2CH_3$ | H | $CF_3$ | 3-$NO_2$ |
| $CO_2CH_3$ | H | $CF_3$ | 4-$NO_2$ |
| $CO_2CH_3$ | H | $CF_3$ | 2-CN |
| $CO_2CH_3$ | H | $CF_3$ | 2,6-$OCH_3$,$OCH_3$ |
| $CO_2CH_3$ | H | $CF_3$ | 2,4,6-Cl,Cl,Cl |
| $CO_2CH_3$ | H | $CF_3$ | 2-Cl,4-$CF_3$ |
| $CO_2CH_3$ | H | $CF_3$ | 3-$N(CH_3)_2$ |

Fortsetzung Tabelle C

| $R^1$ | $R^4$ | $R^2$ | $(R^3)_m$ |
|---|---|---|---|
| $CO_2CH_3$ | H | $OCH_3$ | H |
| $CO_2CH_3$ | H | $OCH_3$ | $3-CH_3$ |
| $CO_2CH_3$ | H | $OCH_3$ | $3-OCH_3$ |
| $CO_2CH_3$ | H | $OCH_3$ | $3-CF_3$ |
| $CO_2CH_3$ | H | $OCH_3$ | 2-F |
| $CO_2CH_3$ | H | $OCH_3$ | 2-Cl |
| $CO_2CH_3$ | H | $OCH_3$ | 3-Cl |
| $CO_2CH_3$ | H | $OCH_3$ | $3-NO_2$ |
| $CO_2CH_3$ | H | $OCH_3$ | $4-NO_2$ |
| $CO_2CH_3$ | H | $OCH_3$ | 2-CN |
| $CO_2CH_3$ | H | $OCH_3$ | $2,6-OCH_3,OCH_3$ |
| $CO_2CH_3$ | H | $OCH_3$ | 2,4,6-Cl,Cl,Cl |
| $CO_2CH_3$ | H | $OCH_3$ | $2-Cl,4-CF_3$ |
| $CO_2CH_3$ | H | $OCH_3$ | $3-N(CH_3)_2$ |
| Cl | H | Cl | H |
| Cl | H | Cl | $3-CH_3$ |
| Cl | H | Cl | $3-OCH_3$ |
| Cl | H | Cl | $3-CF_3$ |
| Cl | H | Cl | 2-F |
| Cl | H | Cl | 2-Cl |
| Cl | H | Cl | 3-Cl |
| Cl | H | Cl | $3-NO_2$ |
| Cl | H | Cl | $4-NO_2$ |
| Cl | H | Cl | 2-CN |
| Cl | H | Cl | $2,6-OCH_3,OCH_3$ |
| Cl | H | Cl | 2,4,6-Cl,Cl,Cl |
| Cl | H | Cl | $2-Cl,4-CF_3$ |
| Cl | H | Cl | $3-N(CH_3)_2$ |

Fortsetzung Tabelle C

| $R^1$ | $R^4$ | $R^2$ | $(R^3)_m$ |
|---|---|---|---|
| Cl | $CH_3$ | Cl | H |
| Cl | $CH_3$ | Cl | 3-$CH_3$ |
| Cl | $CH_3$ | Cl | 3-$OCH_3$ |
| Cl | $CH_3$ | Cl | 3-$CF_3$ |
| Cl | $CH_3$ | Cl | 2-F |
| Cl | $CH_3$ | Cl | 2-Cl |
| Cl | $CH_3$ | Cl | 3-Cl |
| Cl | $CH_3$ | Cl | 3-$NO_2$ |
| Cl | $CH_3$ | Cl | 4-$NO_2$ |
| Cl | $CH_3$ | Cl | 2-CN |
| Cl | $CH_3$ | Cl | 2,6-$OCH_3$,$OCH_3$ |
| Cl | $CH_3$ | Cl | 2,4,6-Cl,Cl,Cl |
| Cl | $CH_3$ | Cl | 2-Cl,4-$CF_3$ |
| Cl | $CH_3$ | Cl | 3-$N(CH_3)_2$ |
| Cl | H | $CH_3$ | H |
| Cl | H | $CH_3$ | 3-$CH_3$ |
| Cl | H | $CH_3$ | 3-$OCH_3$ |
| Cl | H | $CH_3$ | 3-$CF_3$ |
| Cl | H | $CH_3$ | 2-F |
| Cl | H | $CH_3$ | 2-Cl |
| Cl | H | $CH_3$ | 3-Cl |
| Cl | H | $CH_3$ | 3-$NO_2$ |
| Cl | H | $CH_3$ | 4-$NO_2$ |
| Cl | H | $CH_3$ | 2-CN |
| Cl | H | $CH_3$ | 2,6-$OCH_3$,$OCH_3$ |
| Cl | H | $CH_3$ | 2,4,6-Cl,Cl,Cl |
| Cl | H | $CH_3$ | 2-Cl,4-$CF_3$ |
| Cl | H | $CH_3$ | 3-$N(CH_3)_2$ |

Fortsetzung Tabelle C

| $R^1$ | $R^4$ | $R^2$ | $(R^3)_m$ |
|---|---|---|---|
| Cl | H | $CF_3$ | H |
| Cl | H | $CF_3$ | 3-$CH_3$ |
| Cl | H | $CF_3$ | 3-$OCH_3$ |
| Cl | H | $CF_3$ | 3-$CF_3$ |
| Cl | H | $CF_3$ | 2-F |
| Cl | H | $CF_3$ | 2-Cl |
| Cl | H | $CF_3$ | 3-Cl |
| Cl | H | $CF_3$ | 3-$NO_2$ |
| Cl | H | $CF_3$ | 4-$NO_2$ |
| Cl | H | $CF_3$ | 2-CN |
| Cl | H | $CF_3$ | 2,6-$OCH_3$,$OCH_3$ |
| Cl | H | $CF_3$ | 2,4,6-Cl,Cl,Cl |
| Cl | H | $CF_3$ | 2-Cl,4-$CF_3$ |
| Cl | H | $CF_3$ | 3-$N(CH_3)_2$ |
| Cl | H | $OCH_3$ | H |
| Cl | H | $OCH_3$ | 3-$CH_3$ |
| Cl | H | $OCH_3$ | 3-$OCH_3$ |
| Cl | H | $OCH_3$ | 3-$CF_3$ |
| Cl | H | $OCH_3$ | 2-F |
| Cl | H | $OCH_3$ | 2-Cl |
| Cl | H | $OCH_3$ | 3-Cl |
| Cl | H | $OCH_3$ | 3-$NO_2$ |
| Cl | H | $OCH_3$ | 4-$NO_2$ |
| Cl | H | $OCH_3$ | 2-CN |
| Cl | H | $OCH_3$ | 2,6-$OCH_3$,$OCH_3$ |
| Cl | H | $OCH_3$ | 2,4,6-Cl,Cl,Cl |
| Cl | H | $OCH_3$ | 2-Cl,4-$CF_3$ |
| Cl | H | $OCH_3$ | 3-$N(CH_3)_2$ |

Tabelle D

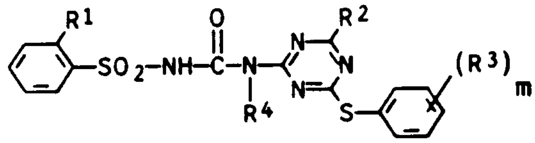

| $R^1$ | $R^4$ | $R^2$ | $(R^3)_m$ |
|---|---|---|---|
| $CO_2CH_3$ | H | Cl | H |
| $CO_2CH_3$ | H | Cl | $3-CH_3$ |
| $CO_2CH_3$ | H | Cl | $3-OCH_3$ |
| $CO_2CH_3$ | H | Cl | $3-CF_3$ |
| $CO_2CH_3$ | H | Cl | 2-F |
| $CO_2CH_3$ | H | Cl | 2-Cl |
| $CO_2CH_3$ | H | Cl | 3-Cl |
| $CO_2CH_3$ | H | Cl | $3-NO_2$ |
| $CO_2CH_3$ | H | Cl | $4-NO_2$ |
| $CO_2CH_3$ | H | Cl | 2-CN |
| $CO_2CH_3$ | H | Cl | $2,6-OCH_3,OCH_3$ |
| $CO_2CH_3$ | H | Cl | 2,4,6-Cl,Cl,Cl |
| $CO_2CH_3$ | H | Cl | $2-Cl,4-CF_3$ |
| $CO_2CH_3$ | H | Cl | $3-N(CH_3)_2$ |
| $CO_2CH_3$ | $CH_3$ | Cl | H |
| $CO_2CH_3$ | $CH_3$ | Cl | $3-CH_3$ |
| $CO_2CH_3$ | $CH_3$ | Cl | $3-OCH_3$ |
| $CO_2CH_3$ | $CH_3$ | Cl | $3-CF_3$ |
| $CO_2CH_3$ | $CH_3$ | Cl | 2-F |
| $CO_2CH_3$ | $CH_3$ | Cl | 2-Cl |
| $CO_2CH_3$ | $CH_3$ | Cl | 3-Cl |
| $CO_2CH_3$ | $CH_3$ | Cl | $3-NO_2$ |
| $CO_2CH_3$ | $CH_3$ | Cl | $4-NO_2$ |
| $CO_2CH_3$ | $CH_3$ | Cl | 2-CN |
| $CO_2CH_3$ | $CH_3$ | Cl | $2,6-OCH_3,OCH_3$ |
| $CO_2CH_3$ | $CH_3$ | Cl | 2,4,6-Cl,Cl,Cl |
| $CO_2CH_3$ | $CH_3$ | Cl | $2-Cl,4-CF_3$ |
| $CO_2CH_3$ | $CH_3$ | Cl | $3-N(CH_3)_2$ |

Fortsetzung Tabelle D

| $R^1$ | $R^4$ | $R^2$ | $(R^3)_m$ |
|---|---|---|---|
| $CO_2CH_3$ | H | $CH_3$ | H |
| $CO_2CH_3$ | H | $CH_3$ | 3-$CH_3$ |
| $CO_2CH_3$ | H | $CH_3$ | 3-$OCH_3$ |
| $CO_2CH_3$ | H | $CH_3$ | 3-$CF_3$ |
| $CO_2CH_3$ | H | $CH_3$ | 2-F |
| $CO_2CH_3$ | H | $CH_3$ | 2-Cl |
| $CO_2CH_3$ | H | $CH_3$ | 3-Cl |
| $CO_2CH_3$ | H | $CH_3$ | 3-$NO_2$ |
| $CO_2CH_3$ | H | $CH_3$ | 4-$NO_2$ |
| $CO_2CH_3$ | H | $CH_3$ | 2-CN |
| $CO_2CH_3$ | H | $CH_3$ | 2,6-$OCH_3$,$OCH_3$ |
| $CO_2CH_3$ | H | $CH_3$ | 2,4,6-Cl,Cl,Cl |
| $CO_2CH_3$ | H | $CH_3$ | 2-Cl,4-$CF_3$ |
| $CO_2CH_3$ | H | $CH_3$ | 3-$N(CH_3)_2$ |
| $CO_2CH_3$ | H | $CF_3$ | H |
| $CO_2CH_3$ | H | $CF_3$ | 3-$CH_3$ |
| $CO_2CH_3$ | H | $CF_3$ | 3-$OCH_3$ |
| $CO_2CH_3$ | H | $CF_3$ | 3-$CF_3$ |
| $CO_2CH_3$ | H | $CF_3$ | 2-F |
| $CO_2CH_3$ | H | $CF_3$ | 2-Cl |
| $CO_2CH_3$ | H | $CF_3$ | 3-Cl |
| $CO_2CH_3$ | H | $CF_3$ | 3-$NO_2$ |
| $CO_2CH_3$ | H | $CF_3$ | 4-$NO_2$ |
| $CO_2CH_3$ | H | $CF_3$ | 2-CN |
| $CO_2CH_3$ | H | $CF_3$ | 2,6-$OCH_3$,$OCH_3$ |
| $CO_2CH_3$ | H | $CF_3$ | 2,4,6-Cl,Cl,Cl |
| $CO_2CH_3$ | H | $CF_3$ | 2-Cl,4-$CF_3$ |
| $CO_2CH_3$ | H | $CF_3$ | 3-$N(CH_3)_2$ |

Fortsetzung Tabelle D

| $R^1$ | $R^4$ | $R^2$ | $(R^3)_m$ |
|---|---|---|---|
| $CO_2CH_3$ | H | $OCH_3$ | H |
| $CO_2CH_3$ | H | $OCH_3$ | 3-$CH_3$ |
| $CO_2CH_3$ | H | $OCH_3$ | 3-$OCH_3$ |
| $CO_2CH_3$ | H | $OCH_3$ | 3-$CF_3$ |
| $CO_2CH_3$ | H | $OCH_3$ | 2-F |
| $CO_2CH_3$ | H | $OCH_3$ | 2-Cl |
| $CO_2CH_3$ | H | $OCH_3$ | 3-Cl |
| $CO_2CH_3$ | H | $OCH_3$ | 3-$NO_2$ |
| $CO_2CH_3$ | H | $OCH_3$ | 4-$NO_2$ |
| $CO_2CH_3$ | H | $OCH_3$ | 2-CN |
| $CO_2CH_3$ | H | $OCH_3$ | 2,6-$OCH_3$,$OCH_3$ |
| $CO_2CH_3$ | H | $OCH_3$ | 2,4,6-Cl,Cl,Cl |
| $CO_2CH_3$ | H | $OCH_3$ | 2-Cl,4-$CF_3$ |
| $CO_2CH_3$ | H | $OCH_3$ | 3-$N(CH_3)_2$ |
| Cl | H | Cl | H |
| Cl | H | Cl | 3-$CH_3$ |
| Cl | H | Cl | 3-$OCH_3$ |
| Cl | H | Cl | 3-$CF_3$ |
| Cl | H | Cl | 2-F |
| Cl | H | Cl | 2-Cl |
| Cl | H | Cl | 3-Cl |
| Cl | H | Cl | 3-$NO_2$ |
| Cl | H | Cl | 4-$NO_2$ |
| Cl | H | Cl | 2-CN |
| Cl | H | Cl | 2,6-$OCH_3$,$OCH_3$ |
| Cl | H | Cl | 2,4,6-Cl,Cl,Cl |
| Cl | H | Cl | 2-Cl,4-$CF_3$ |
| Cl | H | Cl | 3-$N(CH_3)_2$ |

Fortsetzung Tabelle D

| $R^1$ | $R^4$ | $R^2$ | $(R^3)_m$ |
|---|---|---|---|
| Cl | $CH_3$ | Cl | H |
| Cl | $CH_3$ | Cl | 3-$CH_3$ |
| Cl | $CH_3$ | Cl | 3-$OCH_3$ |
| Cl | $CH_3$ | Cl | 3-$CF_3$ |
| Cl | $CH_3$ | Cl | 2-F |
| Cl | $CH_3$ | Cl | 2-Cl |
| Cl | $CH_3$ | Cl | 3-Cl |
| Cl | $CH_3$ | Cl | 3-$NO_2$ |
| Cl | $CH_3$ | Cl | 4-$NO_2$ |
| Cl | $CH_3$ | Cl | 2-CN |
| Cl | $CH_3$ | Cl | 2,6-$OCH_3$,$OCH_3$ |
| Cl | $CH_3$ | Cl | 2,4,6-Cl,Cl,Cl |
| Cl | $CH_3$ | Cl | 2-Cl,4-$CF_3$ |
| Cl | $CH_3$ | Cl | 3-$N(CH_3)_2$ |
| Cl | H | $CH_3$ | H |
| Cl | H | $CH_3$ | 3-$CH_3$ |
| Cl | H | $CH_3$ | 3-$OCH_3$ |
| Cl | H | $CH_3$ | 3-$CF_3$ |
| Cl | H | $CH_3$ | 2-F |
| Cl | H | $CH_3$ | 2-Cl |
| Cl | H | $CH_3$ | 3-Cl |
| Cl | H | $CH_3$ | 3-$NO_2$ |
| Cl | H | $CH_3$ | 4-$NO_2$ |
| Cl | H | $CH_3$ | 2-CN |
| Cl | H | $CH_3$ | 2,6-$OCH_3$,$OCH_3$ |
| Cl | H | $CH_3$ | 2,4,6-Cl,Cl,Cl |
| Cl | H | $CH_3$ | 2-Cl,4-$CF_3$ |
| Cl | H | $CH_3$ | 3-$N(CH_3)_2$ |

Fortsetzung Tabelle D

| $R^1$ | $R^4$ | $R^2$ | $(R^3)_m$ |
|---|---|---|---|
| Cl | H | $CF_3$ | H |
| Cl | H | $CF_3$ | 3-$CH_3$ |
| Cl | H | $CF_3$ | 3-$OCH_3$ |
| Cl | H | $CF_3$ | 3-$CF_3$ |
| Cl | H | $CF_3$ | 2-F |
| Cl | H | $CF_3$ | 2-Cl |
| Cl | H | $CF_3$ | 3-Cl |
| Cl | H | $CF_3$ | 3-$NO_2$ |
| Cl | H | $CF_3$ | 4-$NO_2$ |
| Cl | H | $CF_3$ | 2-CN |
| Cl | H | $CF_3$ | 2,6-$OCH_3$,$OCH_3$ |
| Cl | H | $CF_3$ | 2,4,6-Cl,Cl,Cl |
| Cl | H | $CF_3$ | 2-Cl,4-$CF_3$ |
| Cl | H | $CF_3$ | 3-$N(CH_3)_2$ |
| Cl | H | $OCH_3$ | H |
| Cl | H | $OCH_3$ | 3-$CH_3$ |
| Cl | H | $OCH_3$ | 3-$OCH_3$ |
| Cl | H | $OCH_3$ | 3-$CF_3$ |
| Cl | H | $OCH_3$ | 2-F |
| Cl | H | $OCH_3$ | 2-Cl |
| Cl | H | $OCH_3$ | 3-Cl |
| Cl | H | $OCH_3$ | 3-$NO_2$ |
| Cl | H | $OCH_3$ | 4-$NO_2$ |
| Cl | H | $OCH_3$ | 2-CN |
| Cl | H | $OCH_3$ | 2,6-$OCH_3$,$OCH_3$ |
| Cl | H | $OCH_3$ | 2,4,6-Cl,Cl,Cl |
| Cl | H | $OCH_3$ | 2-Cl,4-$CF_3$ |
| Cl | H | $OCH_3$ | 3-$N(CH_3)_2$ |

Die substituierten Sulfonylharnstoffe I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung

von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 1.002 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.003 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.006 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.002 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 5.005 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.006 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 5.005 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 6.003 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0 kg/ha, vorzugsweise 0,01 bis 1,0 kg/ha.

Die Sulfonylharnstoffe der allgemeinen Formel I, können praktisch alle Entwicklungsstadien einer Pflanze verschiedenartig beeinflussen und werden deshalb auch als Wachstumsregulatoren eingesetzt. Die Wirkungsvielfalt der Verbindungen hängt von verschiedenen Faktoren ab. Maßgeblich sind dabei vor allem

a) Pflanzenart und -sorte,

b) Zeitpunkt der Applikation, bezüglich Entwicklungsstadium der Pflanze und Jahreszeit,

c) Applikationsort und -verfahren (Samenbeize, Bodenbehandlung oder Blattapplikation)

d) klimatische Faktoren, z.B. Temperatur, Niederschlagsmenge, außerdem auch Tageslänge und Lichtintensität

e) Bodenbeschaffenheit (einschließlich Düngung),

f) Formulierung bzw. Anwendungsform des Wirkstoffs und

g) Aufwandmenge und Wirkstoffgehalt der Mittel.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, sind nachstehend einige Beispiele aufgeführt.

A. Mit den wachstumsregulierenden Sulfonylharnstoffen I läßt sich das vegetative Wachstum der Pflanzen stark hemmen, was sich insbesondere in einer Reduzierung des Längenwachstums äußert. Die behandelten Pflanzen weisen demgemäß einen gedrungenen wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Bei Obstgehölzen und anderen Bäumen oder Sträuchern können dadurch kostenintensive Schnittmaßnahmen reduziert werden.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung der Verbindungen I kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den Verbindungen I läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den wachstumsregulierenden Mitteln auf der Basis von Sulfonylharnstoffen I lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Sulfonylharnstoffe der Formel I können Ertragssteigerungen durch Eingriffe in den pflanzlichen Stoffwechsel bzw. durch Förderung oder Hemmung des vegetativen und/oder des generativen Wachstums verursachen.

C. Mit den Sulfonylharnstoffen I lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt-und Sproßachse ist auch für ein gut kontrollierbares Entblättern von Nutzpflanzen z.B. Baumwolle wesentlich.

D. Mit den Sulfonylharnstoffen I kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die Verbindungen I kann die Aufwandmenge stark variiert werden. Bei der Saatgutbehandlung werden im allgemeinen Wirkstoffmengen von 0,001 bis 50 g je Kilogramm Saatgut, vorzugsweise 0,01 bis 1 g, benötigt. Für die Blatt- und Bodenbehandlung sind im allgemeinen Gaben von 0,01 bis 10 kg/ha, bevorzugt 0,1 bis 5 kg/ha ausreichend.

In Anbetracht der Vielseitigkeit der Applikationsmethoden können die erfindungsgemäßen Verbindungen bzw. sie enthaltende Mittel in einer großen Zahl von Kulturplfanzen zur Beseitigung unerwünschter Pflanzen oder zur Beeinflussung des Pflanzenwuchses eingesetzt werden.

Zur Verbreiterung des Wirkungsspektrums und zur Erzielung synergistischer Effekte können die Sulfonylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die neuen Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die in den nachstehenden Beispielen wiedergegebenen Vorschriften wurden unter entsprechender Abwandlung der Ausgangsverbindungen zur Gewinnung weiterer Verbindungen der Formel I benutzt.

Synthesebeispiele

1. Herstellung der Zwischenprodukte III

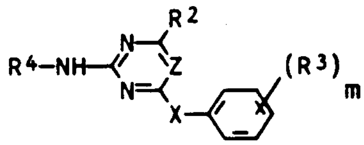

1.1 2-Amino-6-methyl-4-(2-cyano-1-phenoxy)pyrimidin

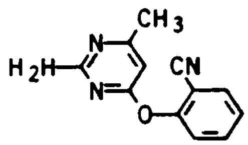

10,0 g (84 mmol) o-Hydroxybenzonitril in 100 ml Methanol wurden unter Rühren bei 60 °C mit 4,7 g Kaliumhydroxid (84 mmol) versetzt, bis eine klare Lösung entstanden war, wonach das Reaktionsgemisch eingeengt wurde. Der Rückstand wurde in 100 ml N-Methyl-2-pyrrolidon aufgenommen, mit 12,0 g (84 mmol) 2-Amino-4-chlor-6-methylpyrimidin versetzt und 6 Stunden bei 140 °C gerührt. Nach dem Abkühlen wurde das Reaktionsgemisch bei 25 °C auf Eis gegeben und der abgeschiedenen Niederschlag wurde isoliert.

Man erhielt so 90 % d. Th. des Produktes mit Fp. 163 - 165 °C.

1.2 2-Amino-4-chlor-6-(2,4-dichlor-1-phenoxy)-1,3,5-triazin

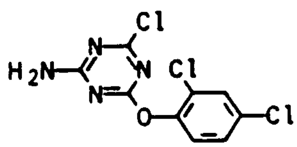

Eine Lösung aus 5,1 g (48,5 mmol) Natriumcarbonat und 150 ml Wasser wurde mit 7,9 g (48,5 mmol) 2,4-Dichlorphenol und 8,0 g (48,5 mmol) 2-Amino-4,6-dichlor-1,3,5-triazin versetzt und 4 Stunden bei 50 °C gerührt. Nach Abkühlen auf 25 °C wurde der Niederschlag abfiltriert und getrocknet; man erhielt so 92 % d. Th. des Produktes.

$^1$H-NMR-Daten (DMSO, int. TMS, 250 MHz): δ 8,32 (breit; NH), 8,24 (breit; NH); 7,83, 7,53 ppm (Multipletts, Aromatenprotonen).

Die in der nachstehenden Tabelle aufgeführten Verbindungen wurden analog erhalten.

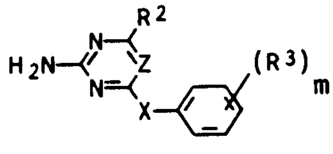

| Nr. | $R^2$ | Z | X | $(R^3)_m$ | Schmelzpunkt (°C) bzw. 1H-NMR-Verschiebung (δ/ppm) des H(5)-Protons am Pyridinring (vs TMS) |
|---|---|---|---|---|---|
| 1.3 | Cl | CH | O | 2-$OCH_3$ | 188-190 |
| 1.4 | Cl | CH | O | 3-$OCH_3$ | 140-142 |
| 1.5 | Cl | CH | O | 4-$OCH_3$ | 6.13($d_6$-DMSO) |
| 1.6 | Cl | CH | O | 2-Cl | 155-158 |
| 1.7 | Cl | CH | O | 3-Cl | 140-141 |
| 1.8 | Cl | CH | O | 2-$NO_2$ | 6.48($d_6$-DMSO) |
| 1.9 | Cl | CH | O | 3-$NO_2$ | 6.40($d_6$-DMSO) |
| 1.10 | Cl | CH | O | 4-$NO_2$ | 6.43($d_6$-DMSO) |
| 1.11 | Cl | CH | O | 2-CN | 6.53($d_6$-DMSO) |
| 1.12 | Cl | CH | O | 3-CN | 204-205 |
| 1.13 | Cl | CH | O | 4-CN | 6.37($d_6$-DMSO) |
| 1.14 | Cl | CH | O | 2-F | 160-165 |
| 1.15 | Cl | CH | O | 3-$CF_3$ | 6.25($CDCl_3$) |
| 1.16 | Cl | CH | O | 2-$OCH_3$, 6-$OCH_3$ | 188-190 |
| 1.17 | Cl | CH | O | 2-Cl, 4-Cl, 6-Cl | 6.36($d_6$-DMSO) |
| 1.18 | $CH_3$ | CH | O | 3-$OCH_3$ | 194-195 |
| 1.19 | $CH_3$ | CH | O | 4-$OCH_3$ | 5.92($CDCl_3$) |
| 1.20 | $CH_3$ | CH | O | 3-CN | 119-120 |
| 1.21 | $CH_3$ | CH | O | 4-CN | 215-217 |
| 1.22 | $CH_3$ | CH | O | 3-$CF_3$ | 6.07($CDCl_3$) |
| 1.23 | $CH_3$ | CH | O | 2-F | 6.13($d_6$-DMSO) |
| 1.24 | $CH_3$ | CH | O | 2-$NO_2$ | 6.20($d_6$-DMSO) |
| 1.25 | $CH_3$ | CH | O | 3-$NO_2$ | 179-180 |
| 1.26 | $CH_3$ | CH | O | 4-$NO_2$ | 219-220 |
| 1.27 | $CH_3$ | CH | O | 2-Cl | 183-184 |
| 1.28 | $CH_3$ | CH | O | 3-Cl | 159-160 |
| 1.29 | $CH_3$ | CH | O | 4-Cl | 219-220 |
| 1.30 | $CH_3$ | CH | O | 2-$OCH_3$, 6-$OCH_3$ | 168-173 |

| Nr. | $R^2$ | Z | X | $(R^3)_m$ | Schmelzpunkt (°C) bzw. $^1H$-NMR-Verschiebung (δ/ppm) des H(5)-Protons am Pyridinring (vs TMS) |
|---|---|---|---|---|---|
| 1.31 | $CH_3$ | CH | O | 2-Cl, 4-Cl, 6-Cl | 130-131 |
| 1.32 | $OCH_3$ | CH | O | 4-$OCH_3$ | 5.36($CDCl_3$) |
| 1.33 | Cl | CH | S | 2-Cl | 6.04($CDCl_3$) |
| 1.34 | Cl | CH | S | 2-$OCH_3$ | 5.74($d_6$-DMSO) |
| 1.35 | Cl | N | O | 2-$OCH_3$ | 230 |
| 1.36 | Cl | N | O | 3-$OCH_3$ | 3.76 [($OCH_3$)$d_6$-DMSO] |
| 1.37 | Cl | N | O | 4-$OCH_3$ | 230 |
| 1.38 | Cl | N | O | 2-Cl | 220-223 |
| 1.39 | Cl | N | O | 3-Cl | 227-228 |
| 1.40 | Cl | N | O | 4-Cl | >230 |
| 1.41 | Cl | N | O | 2-CN | >230 |
| 1.42 | Cl | N | O | 3-CN | >230 |
| 1.43 | Cl | N | O | 4-CN | >230 |
| 1.44 | Cl | CH | O | 2-Cl, 4-Cl | 6,43 ($d_6$-DMSO) |
| 1.45 | Cl | CH | O | 2-Cl, 4-$CF_3$ | 104-106 |
| 1.46 | Cl | CH | O | 3-$N(CH_3)_2$ | 194-204 |
| 1.47 | $CH_3$ | CH | O | 3-$N(CH_3)_2$ | 232-235 |
| 1.48 | $OCH_3$ | CH | O | 2-Cl | 5.53 ($d^6$-DMSO) |
| 1.49 | $OCH_3$ | CH | O | 3-Cl | 112-115 |
| 1.50 | $OCH_3$ | CH | O | 4-Cl | 105-108 |
| 1.51 | $OCH_3$ | CH | O | 2-$OCH_3$ | 104-106 |
| 1.52 | $OCH_3$ | CH | O | 3-$OCH_3$ | 85- 88 |
| 1.53 | $CH_3$ | CH | S | 4-$OCH_3$ | 200-203 |
| 1.54 | F | CH | O | 2-$OCH_3$ | 166 |
| 1.55 | F | CH | O | 3-$OCH_3$ | 115-116 |
| 1.56 | F | CH | O | 4-$OCH_3$ | 195-197 |
| 1.57 | $OCH_3$ | CH | O | 3-$OCH_3$, 4-$OCH_3$, 5-$OCH_3$ | 120-122 |
| 1.58 | $OCH_3$ | CH | O | 4-$OC_2H_5$ | 145-146 |
| 1.59 | $OC_2H_5$ | CH | O | 2-$OCH_3$ | 130-131 |
| 1.60 | $OC_2H_5$ | CH | O | 4-$OC_2H_5$ | 127 |
| 1.61 | $CF_3$ | CH | O | 2-$OCH_3$ | 168-170 |
| 1.62 | $CF_3$ | CH | O | 3-$OCH_3$ | 127-129 |
| 1.63 | $OC_2H_5$ | CH | O | 3-$OCH_3$ | 82 |
| 1.64 | $OCH_3$ | CH | O | 3-$OCH_3$, 5-$OCH_3$ | 85- 86 |
| 1.65 | $OCH_3$ | CH | O | 3-$OCH_3$, 4-$OCH_3$ | 148-149 |
| 1.66 | $CF_3$ | CH | O | 3-$N(CH_3)_2$ | 115-116 |
| 1.67 | $OC_2H_5$ | CH | O | 3-$N(CH_3)_2$ | 122-124 |
| 1.68 | $OCH_3$ | CH | O | 2-$OCH_3$, 3-$OCH_3$ | 5,42 ($CDCl_3$) |
| 1.69 | $OCH_3$ | CH | O | 3,4-($OCH_2O$) | 135-140 |
| 1.70 | $OCH_3$ | CH | O | 2-$OC_2H_5$ | 5,40 ($CDCl_3$) |

| Nr. | $R^2$ | Z | X | $(R^3)_m$ | Schmelzpunkt (°C) bzw. $^1$H-NMR-Verschiebung (δ/ppm) des H(5)-Protons am Pyridinring (vs TMS) |
|---|---|---|---|---|---|
| 1.71 | $OCH_3$ | CH | O | 2-$OCH_3$, 4-$CH_3$ | 5,38 ($CDCl_3$) |
| 1.72 | $OCH_3$ | CH | O | 2-$OCH_3$, 4-(E)-CH=CH-$CH_3$ | 5,39 ($CDCl_3$) |
| 1.73 | $OCH_3$ | CH | O | 2-$OCH_2C_6H_5$ | 75- 78 |
| 1.74 | $OCH_3$ | CH | O | 4-$OCH_2C_6H_5$ | 152-155 |
| 1.75 | Cl | CH | O | 2-$CO_2CH_3$ | 200-201 |
| 1.76 | $OCH_3$ | CH | O | 2-t.-$C_4H_9$, 4-$OCH_3$ | 135-138 |
| 1.77 | $OCH_3$ | CH | O | 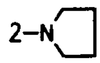 | 117-125 |
| 1.78 | $OCH_3$ | CH | O | 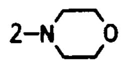 | 174-180 |
| 1.79 | $OCH_3$ | CH | O | 3-$CH_3$, 4-$SCH_3$ | 96- 98 |
| 1.80 | $OCH_3$ | CH | O | 2-Cl, 4-$OCH_3$ | 5,45 ($d_6$-DMSO) |
| 1.81 | $OCH_3$ | CH | O | 4-$SCH_3$ | 5,43 ($d_6$-DMSO) |
| 1.82 | $OCH_3$ | CH | O | 2-$CH_3$, 4-$SCH_3$ | 5.39 ($CDCl_3$) |
| 1.83 | $OCH_3$ | CH | O | 2-$OCH_3$, 4-C(O)$CH_3$ | 5.49 ($CDCl_3$) |
| 1.84 | $OCH_3$ | CH | O | 2-$OC_2H_5$, 4-Cl | 5.42 ($d_6$-DMSO) |

2. Herstellung der Zwischenprodukte V

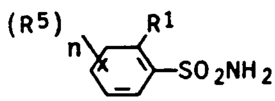

2.1 2-Aminosulfonyl-6-chlor-benzoesäuremethylester

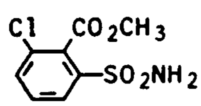

a) 4-Chlor-1,2-benzisothiazol-3-on-1,1-dioxid

504 g (2,02 mol) 6-Chlor-2-aminosulfonyl-benzoesäuremethylester wurden portionsweise zu einer Lösung von 80 g (2,0 mol) Natriumhydroxid in 2,5 l Wasser gegeben, wobei die Temperatur von 25 °C auf 50 °C stieg. Nach 30 Minuten bei dieser Temperatur wurde auf 25 °C abgekühlt, mit Methyl-t-butylether extrahiert und die wäßrige Phase in 2 n Salzsäure eingerührt. Der ausgefallene Niederschlag wurde isoliert, mit Wasser gewaschen und getrocknet. Man erhielt 330 g (75,8 % d. Th.) der Titelverbindung vom Fp.: 210-212 °C.

b) 2-Aminosulfonyl-6-chlor-benzoesäuremethylester

93 g (0,43 mmol) 4-Chlor-1,2-benzisothiazol-3-on-1,1-dioxid wurden in 0,8 l Methanol suspendiert und unter Begasen mit Chlorwasserstoff 3 Stunden unter Rückfluß gerührt. Nach dem Abkühlen auf 20 °C,

Absaugen und Trocknen wurden 56 % d. Th. der Titelverbindung vom Fp. 152-153 °C erhalten. Durch Einengen des Filtrates bei vermindertem Druck und Verreiben des Rückstandes mit Methyl-tert.-butylether, erneutem Abfiltrieren und Trocknen erhielt man 39 % d. Th. einer zweiten Fraktion dieser Verbindung vom Fp. 144 bis 149 °C.

Analog wurden beispielsweise die folgenden Verbindungen hergestellt:

| | 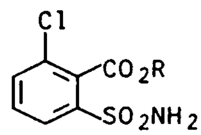 | 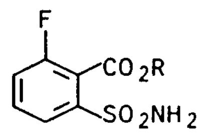 |
|---|---|---|
| R | Fp (°C) | Fp (°C) |
| $C_2H_5$ | 97-101 | 129-131 |
| $n-C_3H_7$ | 111-113 | 104-107 |
| $i-C_3H_7$ | 145-147 | 84- 87 |

2.2 2-Aminosulfonyl-6-fluor-benzoesäuremethylester

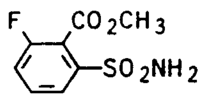

a) 2-Chlorsulfonyl-6-fluor-benzoesäuremethylester

108 g (0,64 mol) 6-Fluoranthranilsäuremethylester und 45 g (0,65 mol) Natriumnitrit in 106 ml Wasser wurden bei 5 °C unter Rühren getrennt aber gleichzeitig innerhalb 1 Stunde so zu 250 ml konz. Salzsäure gegeben, daß die Esterkomponente im Überschuß vorlag. Nach 20 Minuten Rühren bei 5 bis 8 °C wurde die Reaktionsmischung in einem Guß in eine vorbereitete Lösung von 53 g Schwefeldioxid, 1,7 g Kupfer(II)chlorid in wenig Wasser und 200 ml 1,2-Dichlorethan gegeben und weitere 10 Minuten gerührt. Es wurde langsam auf 50 °C erwärmt und unter Einleiten von 46 g Schwefeldioxid 1 1/2 Stunden gerührt. Dann wurde auf 20 °C abgekühlt und während 20 Minuten 5,5 g Chlor unter Rühren eingeleitet. Nach 20 Minuten Rühren wurde die organische Phase abgetrennt, mit Wasser gewaschen und getrocknet. Man erhielt so 65 % d. Th. der Titelverbindung als ein bräunliches Öl. b) 2-Aminosulfonyl-6-fluor-benzoesäuremethylester

42,5 g Ammoniak wurden bei 20 bis 28 °C unter Rühren in eine Mischung von 252,6 g (1 mol) 2-Chlorsulfonyl-6-fluor-benzoesäuremethylester in 700 ml wasserfreiem Tetrahydrofuran eingegast. Nach einer Stunde Rühren bei 25 °C wurde der ausgefallene Niederschlag abgesaugt, in Wasser gelöst und einmal mit Essigester extrahiert. Beim Ansäuern der wäßrigen Phase mit konz. Salzsäure erhielt man 4 % d. Th. 4-Fluor-1,2-benzisothiazol-3-on-1,1-dioxid vom Fp. 210 bis 212 °C.

Das Tetrahydrofuranfiltrat wurde eingeengt, mit Wasser gewaschen, abfiltriert, mit Diethylether nachgewaschen, erneut abfiltriert und getrocknet. Man erhielt 80 % d. Th. der Titelverbindung von Fp. 155 bis 159 °C.

3. Herstellung der Wirkstoffe I

3.1 2-[[[4-Chlor-6-[(3-methoxy)-1-phenoxy]pyrimidin-2-yl]aminocarbonyl]aminosulfonyl]benzoesäuremethylester

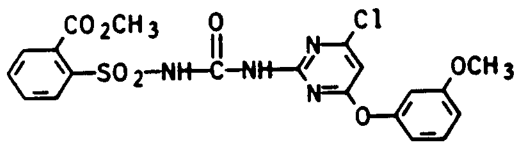

Eine Suspension von 6,0 g (24 mmol) 2-Amino-4-chlor-6-[(3-methoxy)-1-phenoxy]pyrimidin (1.4) in 70 ml Acetonitril wurde bei 25 °C mit einer Lösung von 6,9 g (29 mmol) 2-Isocyanatosulfonylbenzoesäuremethylester in 40 ml Acetonitril versetzt. Nach 8 Stunden Rühren bei 70 °C wurde mit 300 ml Methylenchlorid versetzt, gewaschen, getrocknet und bei 25 °C eingeengt. Das so erhaltene Rohprodukt wurde aus Toluol/Isopropanol umkristallisiert und mit Diethylether gewaschen. Man erhielt so 28 % d. Th. der Titelverbindung mit Fp. 152 - 154 °C. (Wirkstoffbeispiel 1.003). Durch weitere Aufarbeitung der Mutterlauge sowie der Wasch-Phase kann die Ausbeute erhöht werden.

3.2 2-[[[4-Chlor-6-(3-chlor-1-phenoxy)pyrimidin-2-yl]aminocarbonyl]aminosulfonyl]benzoesäuremethylester

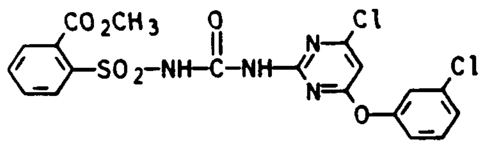

Eine Suspension von 7,0 g (27 mmol) 2-Amino-4-chlor-6-(3-chlor-1-phenoxy)-pyrimidin (1.7) in 80 ml Acetonitril wurde bei Raumtemperatur mit 7,2 g (30 mmol) 2-Isocyanatosulfonylbenzoesäuremethylester versetzt und 2 Stunden bei 70 °C gerührt. Nach Abkühlen auf 25 °C fiel ein Niederschlag aus, der abfiltriert und gewaschen wurde. Nach dem Umkristallisieren aus Essigsäureethylester erhielt man 38 % d. Th. der Titelverbindung mit Fp. 164 - 165 °C. (Wirkstoffbeispiel 1.006). Durch weitere Aufarbeitung der Mutterlauge sowie der Wasch-Phase kann die Ausbeute erhöht werden.

3.3 1-Chlor-2-[[[4-chlor-6-(2,4-dichlor-1-phenoxy)pyrimidin-2-yl]aminocarbonyl]aminosulfonyl)benzol

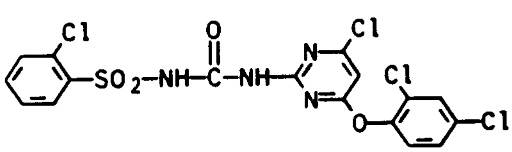

Eine Suspension von 6,0 g (21 mmol) 2-Amino-4-chlor-6-(2,4-dichlor-1-phenoxy)pyrimidin (1.44) in 70 ml wurde bei 25 °C mit 4,9 g (23 mmol) 1-Chlor-2-isocyanatosulfonylbenzol versetzt. Nach 5 Stunden Erwärmen zum Rückfluß und Abkühlen auf 25 °C fiel das Produkt aus. Nach Waschen und Trocknen erhielt man 70 % d. Th. der Titelverbindung mit Fp. 222 - 223 °C. (Wirkstoffbeispiel 2.012)

3.4 1-Chlor-2-[[[4-[(3-methoxy)-1-phenoxy]-6-methylpyridin-2-yl]aminocarbonyl]aminosulfonyl]benzol

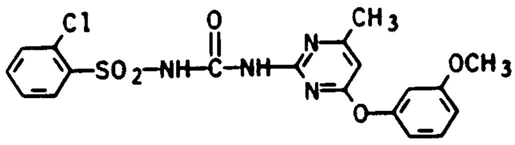

Eine Suspension von 3,6 g (11,2 mmol) 1-Amino-4-[(3-methoxy-1-phenoxy)]-6-methylpyrimidin (1.18) in 50 ml Acetonitril wurde bei 50 °C mit 3,5 g (16,6 mmol) o-Chlorbenzolsulfonylisocyanat versetzt. Nach Einengen der homogenen Lösung auf 5 ml kristallisierte das Produkt. Durch Umkristallisieren mit Diethylether und Trocknen erhielt man 4,8 g 95 % d. Th. der Titelverbindung vom Fp. 156 - 158 °C. (Wirkstoffbeispiel 4.001)

3.5 1-Chlor-2-[[[4-chlor-6-(4-chlor-1-phenoxy)-1,3,5-triazin-2-yl]-aminocarbonyl]aminosulfonyl]-benzol

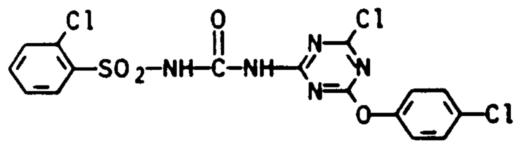

Eine Suspension von 4,0 g (15,6 mmol) 2-Amino-4-chlor-6-(4-chlor-1-phenoxy)-1,3,5-triazin (1.40) in 40 ml Acetonitril wurde mit 1 Spatelspitze 1,4-Diaza(2,2,2)bicyclooctan [DABCO] und 4,2 g (19,3 mmol) o-Chlorbenzolsulfonylisocyanat versetzt und 17 Stunden unter Rückfluß gekocht. Nach dem Abkühlen wurde abfiltriert, mit wenig Acetonitril gewaschen und getrocknet. Man erhielt so 36 % d. Th. der Titelverbindung mit Fp. 187 - 190 °C. (Wirkstoffbeispiel 9.006). Durch weitere Aufarbeitung der Mutterlauge sowie der Wasch-Phase kann die Ausbeute erhöht werden.

Die in den nachstehenden Tabellen 1 - 11 genannten Verbindungen wurden in Analogie zu den beschriebenen Herstellungsvorschriften synthetisiert.

Tabelle 1

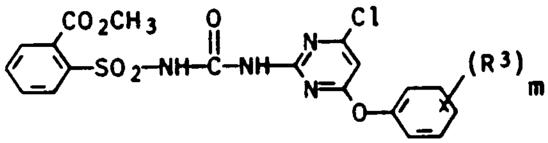

| Nr. | $(R^3)_m$ | Schmelzpunkt (°C) |
|---|---|---|
| 1.001 | H | 145 - 147 |
| 1.002 | $2\text{-}OCH_3$ | 179 - 181 |
| 1.003 | $3\text{-}OCH_3$ | 152 - 154 |
| 1.004 | $4\text{-}OCH_3$ | 170 - 175 |
| 1.005 | 2-Cl | 175 - 177 |
| 1.006 | 3-Cl | 164 - 165 |
| 1.007 | 4-Cl | 175 - 180 |
| 1.008 | $2\text{-}NO_2$ | 142 - 148 |
| 1.009 | $3\text{-}NO_2$ | 200 - 205 |
| 1.010 | $4\text{-}NO_2$ | 213 - 215 |
| 1.011 | 2-CN | 138 - 140 |
| 1.012 | 3-CN | 157 - 165 |
| 1.013 | 4-CN | 208 - 211 |
| 1.014 | 2-F | 133 - 138 |
| 1.015 | $3\text{-}CF_3$ | 198 - 202 |
| 1.016 | $2\text{-}OCH_3$, $6\text{-}OCH_3$ | 120 |
| 1.017 | 2-Cl, 4-Cl; 6-Cl | 210 - 213 |
| 1.018 | $3\text{-}N(CH_3)_2$ | 165 - 170 |
| 1.019 | 2-Cl, $4\text{-}CF_3$ | 72 - 75 |
| 1.020 | $2\text{-}CO_2CH_3$ | 167 - 170 |

Tabelle 2

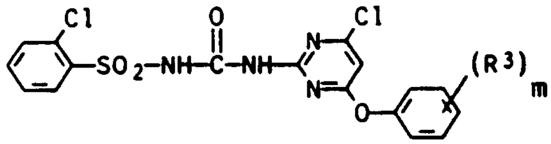

| Nr. | $(R^3)_m$ | Schmelzpunkt (°C) |
|---|---|---|
| 2.001 | 2-Cl | 183 - 185 |
| 2.002 | 3-Cl | 180 - 185 |
| 2.003 | 4-Cl | 211 - 216 |
| 2.004 | $2-NO_2$ | 207 - 211 |
| 2.005 | $3-NO_2$ | 183 - 188 |
| 2.006 | $4-NO_2$ | 210 - 215 |
| 2.007 | 2-CN | 181 - 187 |
| 2.008 | 3-CN | 172 - 176 |
| 2.009 | 4-CN | 188 - 191 |
| 2.010 | 2-F | 175 - 178 |
| 2.011 | $4-OCH_3$ | 159 - 163 |
| 2.012 | 2-Cl, 4-Cl | 222 - 223 |
| 2.013 | $2-OCH_3$, $6-OCH_3$ | 98 |
| 2.014 | 2-Cl, 4-Cl, 6-Cl | 212 - 214 |
| 2.015 | 2-Cl, $4-CF_3$ | 213 - 215 |
| 2.016 | $3-N(CH_3)_2$ | 150 - 155 |

Tabelle 3

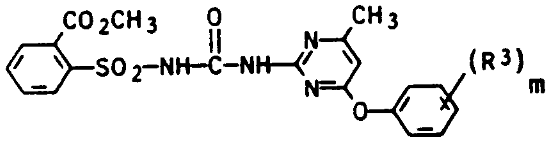

| Nr. | $(R^3)_m$ | Schmelzpunkt (°C) |
|---|---|---|
| 3.001 | 2-$OCH_3$ | 158 - 162 |
| 3.002 | 3-$OCH_3$ | 104 - 107 |
| 3.003 | 2-CN | 157 - 159 |
| 3.004 | 3-CN | 167 - 168 |
| 3.005 | 4-CN | 193 - 195 |
| 3.006 | 2-$OCH_3$, 6-$OCH_3$ | 195 - 197 |
| 3.007 | 2-$NO_2$ | 179 - 180 |
| 3.008 | 3-$NO_2$ | 184 - 185 |
| 3.009 | 4-$NO_2$ | 193 - 196 |
| 3.010 | 3-$CF_3$ | 153 - 156 |
| 3.011 | 3-Cl | 150 - 151 |
| 3.012 | 4-Cl | 193 - 194 |
| 3.013 | 2-F | 152 - 154 |
| 3.014 | 3-$N(CH_3)_2$ | 177 - 180 |
| 3.015 | 2-Cl | 148 - 150 |

Tabelle 4

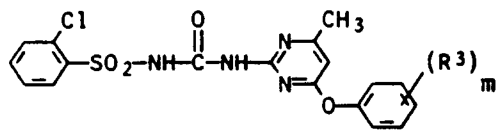

| Nr. | $(R^3)_m$ | Schmelzpunkt (°C) |
|---|---|---|
| 4.001 | $3-OCH_3$ | 156 - 158 |
| 4.002 | $2-OCH_3$, $6-OCH_3$ | 199 - 203 |
| 4.003 | 2-F | 171 - 173 |
| 4.004 | 2-CN | 190 - 195 |
| 4.005 | 3-CN | 206 - 208 |
| 4.006 | 4-CN | 200 - 202 |
| 4.007 | 2-Cl | 188 - 190 |
| 4.008 | 3-Cl | 172 - 173 |
| 4.009 | 4-Cl | 192 - 193 |
| 4.010 | $2-NO_2$ | 204 - 205 |
| 4.011 | $3-NO_2$ | 196 - 198 |
| 4.012 | $3-CF_3$ | 167 - 168 |
| 4.013 | $3-N(CH_3)_2$ | 85 - 90 |

Tabelle 5

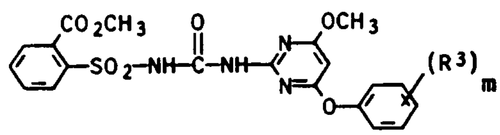

| Nr. | $(R^3)_m$ | Schmelzpunkt (°C) |
|---|---|---|
| 5.001 | $4-OCH_3$ | 146 - 148 |
| 5.002 | 2-Cl | 145 - 150 |
| 5.003 | 3-Cl | 146 - 150 |
| 5.004 | 4-Cl | 156 - 160 |
| 5.005 | $2-OCH_3$ | 104 - 108 |
| 5.006 | $3-OCH_3$ | 148 - 152 |
| 5.007 | $2-OCH_2CH_3$ | 177 - 180 |
| 5.008 | $4-OCH_2CH_3$ | 152 |
| 5.009 | $2-OCH_3, 4-CH_3$ | 167 |
| 5.010 | $3,4-(OCH_3)_2$ | 154 - 155 |
| 5.011 | $2-OCH_3, 4-(E)-CH=CHCH_3$ | 173 - 175 |
| 5.012 | $2,3-(OCH_3)_2$ | 155 |

Tabelle 5 (Fortsetzung)

| Nr. | $(R^3)_m$ | Schmelzpunkt (°C) |
|---|---|---|
| 5.013 | 3,4,5-$(OCH_3)_3$ | 183 - 184 |
| 5.014 | 3,5-$(OCH_3)_2$ | 177 - 178 |
| 5.015 | 3,4-$(OCH_2O)$ | 180 - 183 |
| 5.016 | 2-t.-$C_4H_9$,4-$OCH_3$ | 179 - 182 |
| 5.017 | 4-$OCH_2C_6H_5$ | 145 - 146 |
| 5.018 | 2-$OCH_2C_6H_5$ | 95 - 100 |
| 5.019 | 2- 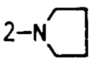 | 163 - 164 |
| 5.020 | 2- 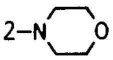 | 196 - 202 |
| 5.021 | 3-$CH_3$,4-$CH_3$ | 176 - 178 |
| 5.022 | 4-$SCH_3$ | 116 - 120 |
| 5.023 | 2-$CH_3$,4-$SCH_3$ | 177 - 179 |
| 5.024 | 2-Cl, 4-$OCH_3$ | 180 |
| 5.025 | 2-$OCH_3$,4-C(O)$CH_3$ | 114 - 116 |
| 5.026 | 2-$OC_2H_5$,4-Cl | 154 - 158 |

Tabelle 6

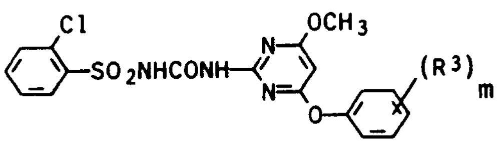

| Nr. | $(R^3)_m$ | Schmelzpunkt (°C) |
|---|---|---|
| 6.001 | 2-Cl | 164 - 170 |
| 6.002 | 3-Cl | 123 - 125 |
| 6.003 | 2-$OCH_3$ | 95 - 100 |
| 6.004 | 3-$OCH_3$ | 75 - 80 |
| 6.005 | 4-Cl | 177 - 182 |
| 6.006 | 3,4-$(OCH_2O)$ | 124 - 134 |
| 6.007 | 2-t.-$C_4H_9$,4-$OCH_3$ | 183 - 187 |
| 6.008 | 4-$OCH_2C_6H_5$ | 145 - 146 |
| 6.009 | 2-$OCH_2C_6H_5$ | 162 |
| 6.010 | 2- 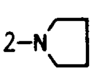 | 173 - 177 |

Tabelle 6 (Fortsetzung)

| Nr. | $(R^3)_m$ | Schmelzpunkt (°C) |
|---|---|---|
| 6.011 | $3-CH_3, 4-SCH_3$ | 163 - 165 |
| 6.012 | $2-Cl, 4-OCH_3$ | 188 |
| 6.013 | $4-SCH_3$ | 145 - 146 |
| 6.014 | $2-CH_3, 4-SCH_3$ | 179 - 184 |
| 6.015 | $2-OCH_2CH_3, 4-Cl$ | 161 - 162 |

Tabelle 7

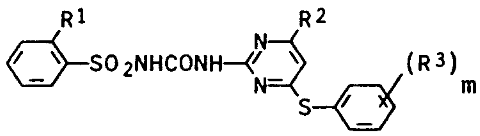

| Nr. | $R^1$ | $R^2$ | $(R^3)_m$ | Schmelzpunkt (°C) |
|---|---|---|---|---|
| 7.001 | $CO_2CH_3$ | Cl | $2-OCH_3$ | 146 - 150 |
| 7.002 | $CO_2CH_3$ | Cl | 2-Cl | 185 - 188 |
| 7.003 | $CO_2CH_3$ | $CH_3$ | $4-OCH_3$ | 193 |
| 7.004 | Cl | Cl | 2-Cl | 200 - 204 |
| 7.005 | Cl | Cl | $2-OCH_3$ | 123 - 127 |
| 7.006 | Cl | $CH_3$ | $4-OCH_3$ | 207 - 208 |

Tabelle 8

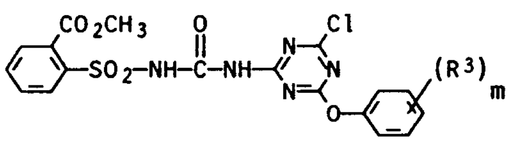

| Nr. | $(R^3)_m$ | Schmelzpunkt (°C) |
|---|---|---|
| 8.001 | $2-OCH_3$ | 184 - 187 |
| 8.002 | $3-OCH_3$ | 162 - 165 |
| 8.003 | $4-OCH_3$ | 210 - 212 |
| 8.004 | 2-Cl | 152 - 156 |
| 8.005 | 3-Cl | 176 - 179 |
| 8.006 | 4-Cl | 165 - 170 |
| 8.007 | 4-CN | 168 - 170 |
| 8.008 | 2-Cl, 4-Cl | 173 - 175 |

Tabelle 9

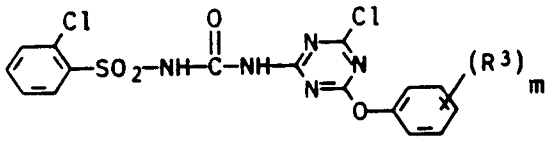

| Nr. | $(R^3)_m$ | Schmelzpunkt (°C) |
|---|---|---|
| 9.001 | 2-$OCH_3$ | 173 - 178 |
| 9.002 | 3-$OCH_3$ | 159 - 162 |
| 9.003 | 4-$OCH_3$ | 162 - 167 |
| 9.004 | 2-Cl | 189 - 193 |
| 9.005 | 3-Cl | 165 - 169 |
| 9.006 | 4-Cl | 187 - 190 |
| 9.007 | 2-CN | 174 - 176 |
| 9.008 | 3-CN | 186 - 188 |

Tabelle 10

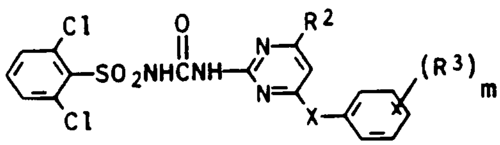

| Nr. | $R^2$ | X | $(R^3)_m$ | Schmelzpunkt (°C) |
|---|---|---|---|---|
| 10.001 | Cl | S | 2-$OCH_3$ | 209 - 211 |
| 10.002 | Cl | O | 4-Cl | >230 |
| 10.003 | Cl | O | 3-Cl | 215 - 225 |
| 10.004 | Cl | O | 2-Cl | 195 - 198 |
| 10.005 | Cl | O | 4-$OCH_3$ | 210 - 215 |
| 10.006 | Cl | O | 3-$OCH_3$ | 182 - 188 |
| 10.007 | Cl | O | 2-$OCH_3$ | 155 - 160 |
| 10.008 | $OCH_3$ | O | 4-$OCH_3$ | 155 - 158 |

Tabelle 11

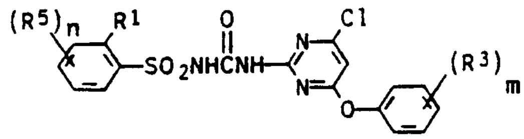

| Nr. | $R^1$ | $(R^5)_n$ | $(R^3)_m$ | Schmelzpunkt (°C) |
|---|---|---|---|---|
| 11.001 | $CO_2(i\text{-}C_3H_7)$ | H | 3-$OCH_3$ | 130 - 133 |
| 11.002 | $CO_2CH_3$ | 5-F | 3-$OCH_3$ | 156 - 158 |
| 11.003 | $CO_2CH_3$ | 5-F | 2-$OCH_3$ | 125 - 127 |
| 11.004 | $CO_2CH_3$ | 3-Cl | 3-$OCH_3$ | 132 |
| 11.005 | $CO_2CH_3$ | 3-Cl | 4-$OCH_3$ | 192 - 194 |
| 11.006 | $CO_2CH_3$ | 5-F | 4-$OCH_3$ | 178 - 179 |
| 11.007 | $CO_2(CH_2)_2Cl$ | H | 3-$OCH_3$ | 148 |
| 11.008 | $CO_2(CH_2)_2Cl$ | H | 2-$OCH_3$ | 166 - 167 |
| 11.009 | $CO_2(CH_2)_2Cl$ | H | 4-$OCH_3$ | 172 - 173 |
| 11.010 | $CO_2(i\text{-}C_3H_7)$ | H | 2-$OCH_3$ | 143 - 145 |
| 11.011 | $CO_2CH_3$ | 3-Cl | 2-$OCH_3$ | 168 - 170 |
| 11.012 | $CO_2(CH_2)_2CH_3$ | H | 2,6-$(OCH_3)_2$ | 70 - 80 |

Tabelle 12

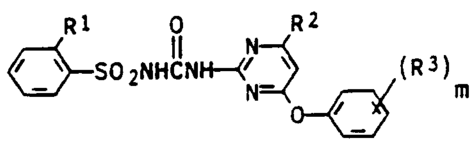

| Nr. | $R^1$ | $R^2$ | $(R^3)_m$ | Schmelzpunkt (°C) |
|---|---|---|---|---|
| 12.001 | $CO_2CH_3$ | F | 2-$OCH_3$ | 161 |
| 12.002 | $CO_2CH_3$ | F | 3-$OCH_3$ | 143 |
| 12.003 | $CO_2CH_3$ | F | 4-$OCH_3$ | 167 - 170 |
| 12.004 | $CO_2CH_3$ | $CF_3$ | 2-$OCH_3$ | 115 |
| 12.005 | $CO_2CH_3$ | $CF_3$ | 3-$OCH_3$ | 177 - 178 |
| 12.006 | $CO_2CH_3$ | $CF_3$ | 4-$OCH_3$ | 188 |
| 12.007 | $CO_2CH_3$ | $CF_3$ | 3-$N(CH_3)_2$ | 176 - 177 |
| 12.008 | $CO_2CH_3$ | $OC_2H_5$ | 2-$OCH_3$ | 109 |
| 12.009 | $CO_2CH_3$ | $OC_2H_5$ | 3-$OCH_3$ | 71 - 75 |
| 12.010 | $CO_2CH_3$ | $OC_2H_5$ | 4-$OCH_3$ | 128 - 131 |
| 12.011 | $CO_2CH_3$ | $OC_2H_5$ | 3-$N(CH_3)_2$ | 142 |

Anwendungsbeispiele

Die heribzide Wirkung der Sulfonylharnstoffe der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen wurden nach Arten getrennt eingesät.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Zum Zweck der Nachauflaufbehandlung wurden entweder direkt gesäte oder in den gleichen Gefäßen aufgewachsene Pflanzen ausgewählt oder sie wurden erst als Keimpflanzen getrennt angezogen und einige Tage vor der Behandlung in die Versuchsgefäße verpflanzt.

Je nach Wuchsform werden die Testpflanzen bei einer Wuchshöhe von 3 - 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung betrug 0,5, 0,25 bzw. 0,125 kg/ha a.S.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufanwendung betrug 0,125 kg/ha a.S.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

In den Gewächshausversuchen wurden Pflanzen der folgenden Arten verwendet:

Pflanzenliste

| Lateinischer Name | Deutscher Name |
|---|---|
| Amaranthus retroflexus | Zurückgekrümmter Fuchsschwanz |
| Chrysanthemum corinarium | Kronenwucherblume |
| Cyperus iria | - |
| Galium aparine | Klettenlabkraut |
| Helianthus annuus | Sonnenblume |
| Sesbania exaltata | Turibaum |
| Stellaria media | Vogelsternmiere |
| Triticum aestivum | Sommerweizen |

Mit 0,5 bzw. 0,25 kg/ha a. S. (aktive Substanz) im Nachauflaufverfahren eingesetzt, lassen sich mit den Beispielen 1.002, 1.003 und 1.006 unerwünschte Pflanzen sehr gut bekämpfen.

Mit 0,125 kg/ha a. S. lassen sich im Vorauflauf- und im Nachauflaufverfahren unerwünschte breitblättrige Pflanzen durch die Verbindungen 5.005 und 6.003 sehr gut bekämpfen, ohne daß die Beispielkultur Weizen nennenswert geschädigt wird.

## Patentansprüche

## Patentansprüche für folgende Vertragsstaaten : BE, CH, DE, FR, GB, IT, LI

**1.** Substituierte Sulfonylharnstoffe der allgemeinen Formel I

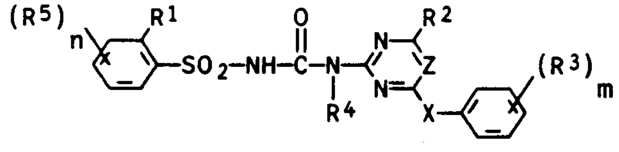

I

in der die Substituenten und die Indices folgende Bedeutung haben:

X Sauerstoff oder Schwefel;

Z Stickstoff oder eine Methingruppe = CH-;

- $R^1$ Halogen, $C_1$-$C_4$-Alkoxycarbonyl, welches ein bis drei der folgenden Reste tragen kann: Halogen, $C_1$-$C_4$-Alkoxy, $C_1$-$C_4$-Halogenalkoxy, $C_1$-$C_4$-Alkylthio und/oder $C_1$-$C_4$-Halogenalkylthio;
  $C_1$-$C_3$-Alkoxy, welches ein bis drei der folgenden Reste tragen kann: Halogen, $C_1$-$C_4$-Alkoxy, $C_1$-$C_4$-Halogenalkoxy, $C_1$-$C_4$-Alkylthio und/oder $C_1$-$C_4$-Halogenalkylthio;
  oder ein Rest $-CONR^6R^7$, worin
- $R^6$ Wasserstoff, eine $C_1$-$C_8$-Alkylgruppe oder eine $C_1$-$C_6$-Alkoxygruppe und
- $R^7$ Wasserstoff oder eine $C_1$-$C_8$-Alkylgruppe bedeuten;
- $R^2$ Halogen; $C_1$-$C_4$-Alkoxy, oder $C_1$-$C_4$-Alkyl, welche ein bis drei der folgenden Reste tragen können: Halogen, $C_1$-$C_4$-Alkoxy und/oder $C_1$-$C_4$-Alkylthio;
- $R^3$ $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio, Halogen, Cyano, Nitro, Amino, Mono-$C_1$-$C_4$-alkylamino, Di-$C_1$-$C_4$-alkylamino, $C_2$-$C_6$-Alkenyl, $C_1$-$C_4$-Alkoxycarbonyl, $C_1$-$C_4$-Alkanoyl oder Benzyl; ein 5- oder 6-gliedriger über sein Stickstoffatom gebundener gesättigter Heterocyclus, enthaltend neben Methylengruppen ein Stickstoffatom, wobei dieser Cyclus außerdem ein Sauerstoff- oder ein Schwefelatom enthalten kann,
  sowie an benachbarte Ringpositionen gebundenes -OCRR'O-, wobei R und R' Wasserstoff oder $C_1$-$C_4$-Alkyl bedeuten;
- $R^4$ Wasserstoff oder $C_1$-$C_4$-Alkyl;
- $R^5$ Halogen, $C_1$-$C_4$-Alkyl, $C_1$-$C_4$-Alkoxy, $C_1$-$C_4$-Alkylthio oder $C_1$-$C_4$-Halogenalkyl;
- m 0 bis 3, oder 0 bis 5, wenn $R^3$ Halogen bedeutet, wobei die Reste $R^3$ verschieden sein können, wenn m 2 oder 3 bedeutet;
- n 0 bis 2, wobei die Reste $R^5$ verschieden sein können, wenn n 2 bedeutet;

sowie deren umweltverträgliche Salze.

**2.** Verfahren zur Herstellung der substituierten Sulfonylharnstoffe I gemäß Anspruch 1, dadurch gekennzeichnet, daß man ein Sulfonylisocyanat II

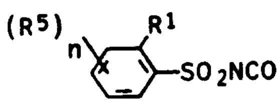

II

in an sich bekannter Weise in einem inerten organischen Lösungsmittel mit einem 2-Amino-heteroaryletherderivat III

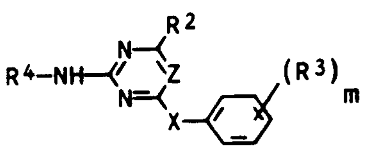

III

umsetzt.

**3.** Verfahren zur Herstellung der substituierten Sulfonylharnstoffe I gemäß Anspruch 1, dadurch gekennzeichnet, daß man ein Carbamat der Formel IV

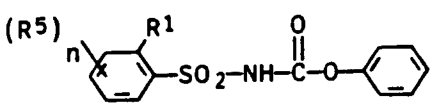

IV

in an sich bekannter Weise in einem inerten organischen Lösungsmittel bei 0 bis 120 °C mit einem 2-Amino-heteroarylether III umsetzt.

**4.** Verfahren zur Herstellung der substituierten Sulfonylharnstoffen I gemäß Anspruch 1, dadurch gekennzeichnet, daß man ein entsprechendes Sulfonamid der Formel V

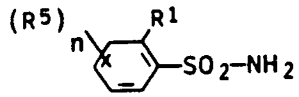

V

in an sich bekannter Weise in einem inerten organischen Lösungsmittel mit einem Phenylcarbamat VI

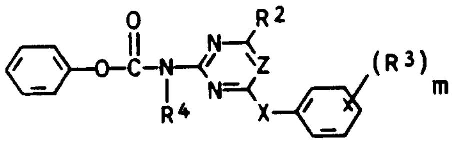

VI

umsetzt.

5. Herbizides Mittel, enthaltend einen substituierten Sulfonylharnstoff I gemäß Anspruch 1 oder sein Salz sowie hierfür übliche Formulierungshilfsstoffe.

6. Verfahren zur Bekämpfung unerwünschten Pflanzenwuchses, dadurch gekennzeichnet, daß man eine herbizid wirksame Menge eines substituierten Sulfonylharnstoffs I gemäß Anspruch 1 oder eines seiner Salze auf die Pflanzen und/oder ihren Lebensraum einwirken läßt.

7. Verwendung der substituierten Sulfonylharnstoffe I gemäß Anspruch 1 als herbizide Mittel.

8. Mittel zur Beeinflussung des Pflanzenwuchses, enthaltend einen substituierten Sulfonylharnstoff I gemäß Anspruch 1 oder sein Salz sowie hierfür übliche Formulierungshilfsstoffe.

9. Verfahren zur Beeinflussung des Pflanzenwuchses, dadurch gekennzeichnet, daß man eine den Wuchs regulierende Menge eines substituierten Sulfonylharnstoffs I gemäß Anspruch 1 oder eines seiner Salze auf die Pflanzen und/oder ihren Lebensraum einwirken läßt.

**Patentansprüche für folgenden Vertragsstaat: ES**

1. Verfahren zur Herstellung von Sulfonylharnstoffen der allgemeinen Formel I

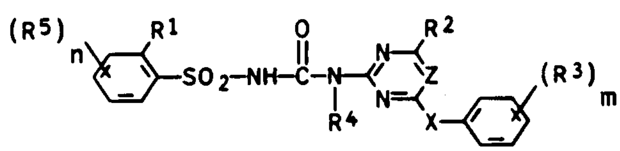

I

in der die Substituenten und die Indices folgende Bedeutung haben:

X Sauerstoff oder Schwefel;

Z Stickstoff oder eine Methingruppe = CH-;

$R^1$ Halogen, $C_1$-$C_4$-Alkoxycarbonyl, welches ein bis drei der folgenden Reste tragen kann: Halogen, $C_1$-$C_4$-Alkoxy, $C_1$-$C_4$-Halogenalkoxy, $C_1$-$C_4$-Alkylthio und/oder $C_1$-$C_4$-Halogenalkylthio;
$C_1$-$C_3$-Alkoxy, welches ein bis drei der folgenden Reste tragen kann: Halogen, $C_1$-$C_4$-Alkoxy, $C_1$-$C_4$-Halogenalkoxy, $C_1$-$C_4$-Alkylthio und/oder $C_1$-$C_4$-Halogenalkylthio;
oder ein Rest -$CONR^6R^7$, worin

$R^6$ Wasserstoff, eine $C_1$-$C_8$-Alkylgruppe oder eine $C_1$-$C_6$-Alkoxygruppe und

$R^7$ Wasserstoff oder eine $C_1$-$C_8$-Alkylgruppe bedeuten;

$R^2$ Halogen; $C_1$-$C_4$-Alkoxy, oder $C_1$-$C_4$-Alkyl, welche ein bis drei der folgenden Reste tragen können: Halogen, $C_1$-$C_4$-Alkoxy und/oder $C_1$-$C_4$-Alkylthio;

$R^3$ $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio, Halogen, Cyano, Nitro, Amino, Mono-$C_1$-$C_4$-alkylamino, Di-$C_1$-$C_4$-alkyla-

mino, $C_2$-$C_6$-Alkenyl, $C_1$-$C_4$-Alkoxycarbonyl, oder Benzyl;
ein 5- oder 6-gliedriger über sein Stickstoffatom gebundener gesättigter Heterocyclus, enthaltend neben Methylengruppen ein Stickstoffatom, wobei dieser Cyclus außerdem ein Sauerstoff- oder ein Schwefelatom enthalten kann,
sowie an benachbarte Ringpositionen gebundenes -OCRR'O-, wobei R und R' Wasserstoff oder $C_1$-$C_4$-Alkyl bedeuten;

$R^4$ Wasserstoff oder $C_1$-$C_4$-Alkyl;

$R^5$ Halogen, $C_1$-$C_4$-Alkyl, $C_1$-$C_4$-Alkoxy, $C_1$-$C_4$-Alkylthio oder $C_1$-$C_4$-Halogenalkyl;

m 0 bis 3, oder 0 bis 5, wenn $R^3$ Halogen bedeutet, wobei die Reste $R^3$ verschieden sein können, wenn m 2 oder 3 bedeutet;

n 0 bis 2, wobei die Reste $R^5$ verschieden sein können, wenn n 2 bedeutet;

sowie deren umweltverträgliche Salze, dadurch gekennzeichnet, daß man ein Sulfonylisocyanat II

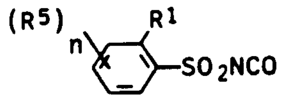

II

in an sich bekannter Weise in einem inerten organischen Lösungsmittel mit einem 2-Amino-heteroaryletherderivat III

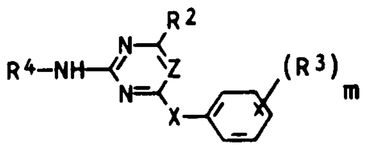

III

umsetzt.

2. Verfahren zur Herstellung der substituierten Sulfonylharnstoffe I gemäß Anspruch 1, dadurch gekennzeichnet, daß man ein Carbamat der Formel IV

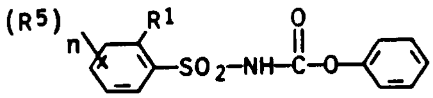

IV

in an sich bekannter Weise in einem inerten organischen Lösungsmittel bei 0 bis 120°C mit einem 2-Amino-heteroarylether III umsetzt.

3. Verfahren zur Herstellung der substituierten Sulfonylharnstoffen I gemäß Anspruch 1, dadurch gekennzeichnet, daß man ein entsprechendes Sulfonamid der Formel V

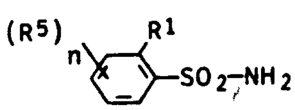

V

in an sich bekannter Weise in einem inerten organischen Lösungsmittel mit einem Phenylcarbamat VI

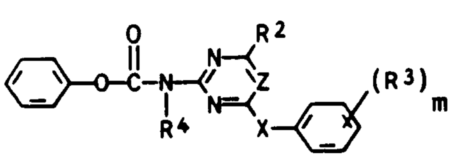

VI

umsetzt.

**4.** Herbizides Mittel, enthaltend einen substituierten Sulfonylharnstoff I gemäß Anspruch 1 oder sein Salz sowie hierfür übliche Formulierungshilfsstoffe.

**5.** Verfahren zur Bekämpfung unerwünschten Pflanzenwuchses, dadurch gekennzeichnet, daß man eine herbizid wirksame Menge eines substituierten Sulfonylharnstoffs I gemäß Anspruch 1 oder eines seiner Salze auf die Pflanzen und/oder ihren Lebensraum einwirken läßt.

**6.** Verwendung der substituierten Sulfonylharnstoffe I gemäß Anspruch 1 als herbizide Mittel.

**7.** Mittel zur Beeinflussung des Pflanzenwuchses, enthaltend einen substituierten Sulfonylharnstoff I gemäß Anspruch 1 oder sein Salz sowie hierfür übliche Formulierungshilfsstoffe.

**8.** Verfahren zur Beeinflussung des Pflanzenwuchses, dadurch gekennzeichnet, daß man eine den Wuchs regulierende Menge eines substituierten Sulfonylharnstoffs I gemäß Anspruch 1 oder eines seiner Salze auf die Pflanzen und/oder ihren Lebensraum einwirken läßt.

**Claims**
**Claims for the following Contracting States: BE, CH, DE, FR, GB, IT, LI**

**1.** Substituted sulfonylureas of the general formula I

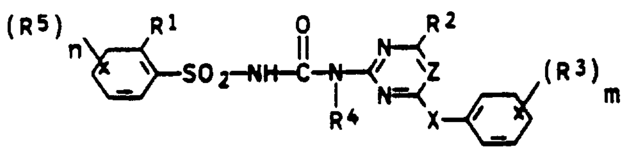

I

where the substituents and indices have the following meanings:

X is oxygen or sulfur;
Z is nitrogen or methine (=CH-);
$R^1$ is halogen, $C_1$-$C_4$-alkoxycarbonyl which may carry from one to three of the following radicals: halogen, $C_1$-$C_4$-alkoxy, $C_1$-$C_4$-haloalkoxy, $C_1$-$C_4$-alkylthio and/or $C_1$-$C_4$-haloalkylthio; $C_1$-$C_3$-alkoxy which may carry from one to three of the following radicals: halogen, $C_1$-$C_4$-alkoxy, $C_1$-$C_4$-haloalkoxy, $C_1$-$C_4$-alkylthio and/or $C_1$-$C_4$-haloalkylthio; or a radical -CONR$^6$R$^7$, where $R^6$ is hydrogen, $C_1$-$C_8$-alkyl or $C_1$-$C_6$-alkoxy and $R^7$ is hydrogen or $C_1$-$C_8$-alkyl;
$R^2$ is halogen; $C_1$-$C_4$-alkoxy or $C_1$-$C_4$-alkyl which may each carry from one to three of the following radicals: halogen, $C_1$-$C_4$-alkoxy and/or $C_1$-$C_4$-alkylthio;
$R^3$ is $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, $C_1$-$C_4$-haloalkylthio, halogen, cyano, nitro, amino, mono-$C_1$-$C_4$-alkylamino, di-$C_1$-$C_4$-alkylamino, $C_2$-$C_6$-alkenyl, $C_1$-$C_4$-alkoxycarbonyl, $C_1$-$C_4$-alkanoyl or benzyl; a 5- or 6-membered saturated heterocycle which is attached by its nitrogen atom and which, besides methylene and a nitrogen, may also contain an oxygen or sulfur atom; or, bonded to adjacent ring positions, -OCRR'O-, where R and R' are each hydrogen or $C_1$-$C_4$-alkyl;
$R^4$ is hydrogen or $C_1$-$C_4$-alkyl;
$R^5$ is halogen, $C_1$-$C_4$-alkyl, $C_1$-$C_4$-alkoxy, $C_1$-$C_4$-alkylthio or $C_1$-$C_4$-haloalkyl;
m is from 0 to 3 or, when $R^3$ is halogen, from 0 to 5, differences among the $R^3$ radicals being possible when m is 2 or 3; and
n is from 0 to 2, a difference between the $R^5$ radicals being possible when n is 2;

and environmentally acceptable salts thereof.

**2.** A process for preparing the substituted sulfonylureas I of claim 1, which comprises reacting a sulfonyl isocyanate II

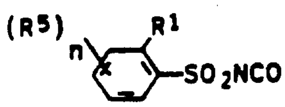

II

in a conventional manner in an inert organic solvent with a 2-aminohetaryl ether derivative III

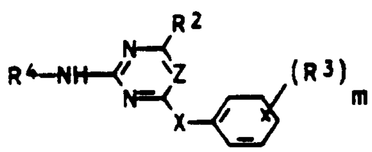

III .

3. A process for preparing the substituted sulfonylureas I of claim 1, which comprises reacting a carbamate of the formula IV

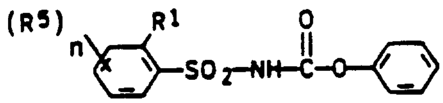

IV

in a conventional manner in an inert organic solvent at from 0 to 120 °C with a 2-aminohetaryl ether III.

4. A process for preparing the substituted sulfonylureas I of claim 1, which comprises reacting a corresponding sulfonamide of the formula V

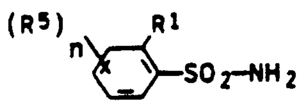

V

in a conventional manner in an inert organic solvent with a phenyl carbamate VI

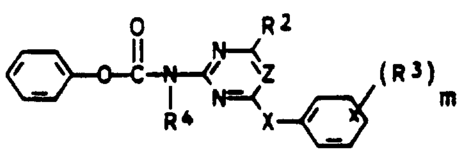

VI .

5. A herbicidal composition comprising a substituted sulfonylurea I as claimed in claim 1, or a salt thereof, and conventional formulation auxiliaries therefor.

6. A method of controlling unwanted plant growth, which comprises treating the plants and/or their habitat with a herbicidally effective amount of a substituted sulfonylurea I as claimed in claim 1, or of a salt thereof.

7. The use of substituted sulfonylureas I as claimed in claim 1 as herbicides.

8. A plant growth regulator comprising a substituted sulfonylurea I as claimed in claim 1, or a salt thereof, and conventional formulation auxiliaries therefor.

9. A method for influencing plant growth, which comprises treating the plants and/or their habitat with a growth-regulating amount of a substituted sulfonylurea I as claimed in claim 1, or of a salt thereof.

**Claims for the following Contracting State : ES**

**1.** A process for preparing sulfonylureas of the general formula I

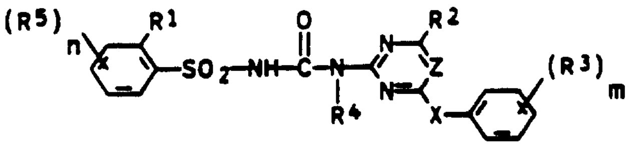

I

where the substituents and indices have the following meanings:

X is oxygen or sulfur;

Z is nitrogen or methine (=CH-);

$R^1$ is halogen, $C_1$-$C_4$-alkoxycarbonyl which may carry from one to three of the following radicals: halogen, $C_1$-$C_4$-alkoxy, $C_1$-$C_4$-haloalkoxy, $C_1$-$C_4$-alkylthio and/or $C_1$-$C_4$-haloalkylthio; $C_1$-$C_3$-alkoxy which may carry from one to three of the following radicals: halogen, $C_1$-$C_4$-alkoxy, $C_1$-$C_4$-haloalkoxy, $C_1$-$C_4$-alkylthio and/or $C_1$-$C_4$-haloalkylthio; or a radical $-CONR^6R^7$, where $R^6$ is hydrogen, $C_1$-$C_8$-alkyl or $C_1$-$C_6$-alkoxy and $R^7$ is hydrogen or $C_1$-$C_8$-alkyl;

$R^2$ is halogen; $C_1$-$C_4$-alkoxy or $C_1$-$C_4$-alkyl which may each carry from one to three of the following radicals: halogen, $C_1$-$C_4$-alkoxy and/or $C_1$-$C_4$-alkylthio;

$R^3$ is $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, $C_1$-$C_4$-haloalkylthio, halogen, cyano, nitro, amino, mono-$C_1$-$C_4$-alkylamino, di-$C_1$-$C_4$-alkylamino, $C_2$-$C_6$-alkenyl, $C_1$-$C_4$-alkoxycarbonyl or benzyl; a 5- or 6-membered saturated heterocycle which is attached by its nitrogen atom and which, besides methylene and a nitrogen, may also contain an oxygen or sulfur atom; or, bonded to adjacent ring positions, -OCRR'O-, where R and R' are each hydrogen or $C_1$-$C_4$-alkyl;

$R^4$ is hydrogen or $C_1$-$C_4$-alkyl;

$R^5$ is halogen, $C_1$-$C_4$-alkyl, $C_1$-$C_4$-alkoxy, $C_1$-$C_4$-alkylthio or $C_1$-$C_4$-haloalkyl;

m is from 0 to 3 or, when $R^3$ is halogen, from 0 to 5, differences among the $R^3$ radicals being possible when m is 2 or 3; and

n is from 0 to 2, a difference between the $R^5$ radicals being possible when n is 2;

and environmentally acceptable salts thereof, which comprises reacting a sulfonyl isocyanate II

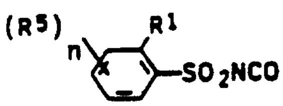

II

in a conventional manner in an inert organic solvent with a 2-aminohetaryl ether derivative III

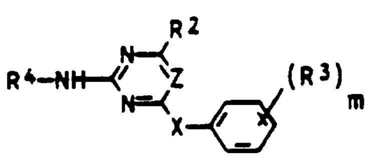

III .

**2.** A process for preparing the substituted sulfonylureas I as set forth in claim 1, which comprises reacting a carbamate of the formula IV

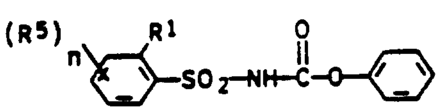

IV

in a conventional manner in an inert organic solvent at from 0 to 120 °C with a 2-aminohetaryl ether III.

3. A process for preparing the substituted sulfonylureas I as set forth in claim 1, which comprises reacting a corresponding sulfonamide of the formula V

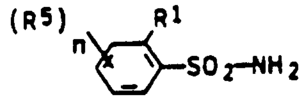

V

in a conventional manner in an inert organic solvent with a phenyl carbamate VI

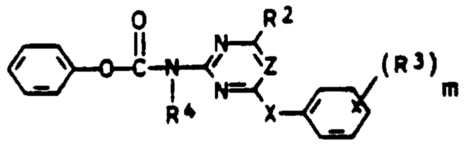

VI .

4. A herbicidal composition comprising a substituted sulfonylurea I as set forth in claim 1, or a salt thereof, and conventional formulation auxiliaries therefor.

5. A method of controlling unwanted plant growth, which comprises treating the plants and/or their habitat with a herbicidally effective amount of a substituted sulfonylurea I as set forth in claim 1, or of a salt thereof.

6. The use of substituted sulfonylureas I as set forth in claim 1 as herbicides.

7. A plant growth regulator comprising a substituted sulfonylurea I as set forth in claim 1, or a salt thereof, and conventional formulation auxiliaries therefor.

8. A method for influencing plant growth, which comprises treating the plants and/or their habitat with a growth-regulating amount of a substituted sulfonylurea I as set forth in claim 1, or of a salt thereof.

**Revendications**
**Revendications pour les Etats contractants suivants : BE, CH, DE, FR, GB, IT, LI**

1. Sulfonylurées substituées de la formule générale I

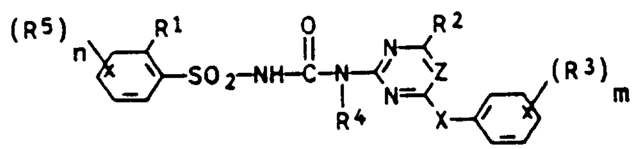

I

dans laquelle les substituants et les indices ont les significations suivantes :

- X représente oxygène ou soufre
- Z azote ou un groupe méthine = CH-
- $R^1$ halogène, alcoxy carbonyle en C1-C4 qui peut porter un à trois des restes suivants : halogène, alcoxy en C1-C4, halogénoalcoxy en C1-C4, alkylthio en C1-C4 et/ou halogénoalkylthio en C1-C4, alcoxy en C1-C3, qui peut porter un à trois des restes suivants : halogène, alcoxy en C1-C4, halogénalcoxy en C1-C4, alkylthio en C1-C4 et/ou halogénalkylthio en C1-C4;

ou un reste $-CONR^6R^7$, où

- $R^6$ hydrogène, un groupe alkyle en C1-C8 ou un groupe alcoxy en C1-C6 et
- $R^7$ hydrogène ou un groupe alkyle en C1-C8;
- $R^2$ halogène, alcoxy en C1-C4 ou alkyle en C1-C4 qui peuvent porter un à trois des restes suivants : halogène, alcoxy en C1-C4 et/ou alkylthio en C1-C4
- $R^3$ alkyle en C1-C4, alcoxy en C1-C4, alkylthio en C1-C4, halogénalkyle en C1-C4, halogénalcoxy en C1-C4, halogénalkylthio en C1-C4, halogène, cyano, nitro, amino, mono-alkyle en C1-C4-amino, di-alkyle en C1-C4-amino, alcényle en C2-C6, alcoxycarbonyle en C1-C4, alcanoyle en C1-C4 ou benzyle;
un hétérocycle saturé, lié par son atome d'azote, à 5 ou 6 chainons, contenant outre des groupes méthylène un atome d'azote, ce cycle pouvant en outre contenir un atome d'oxygène ou de soufre
ainsi que -OCRR'O- liés sur des positions voisines du noyau, R et R' signifiant hydrogène ou alkyle en C1-C4

$R^4$ hydrogène ou alkyle en C1-C4

$R^5$ halogène, alkyle en C1-C4, alcoxy en C1-C4, alkylthio en C1-C4 ou halogénalkyle en C1-C4;

m 0 à 3 ou 0 à 5 quand $R^3$ représente halogène, les restes $R^3$ pouvant être différents quand m représente 2 ou 3

n 0 à 2, les restes $R^5$ pouvant être différents quand n = 2

ainsi que leurs sels acceptables pour l'environnement.

**2.** Procédé de préparation des sulfonylurées substituées de la formule générale I selon la revendication 1, caractérisé par le fait que l'on fait réagir un sulfonylisocyanate II

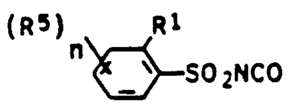

II

de manière connue en soi, dans un solvant organique inerte avec un dérivé de 2-amino-hétéroaryléther III

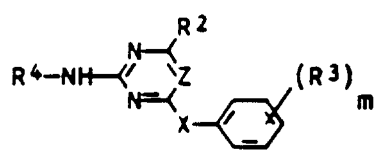

III

**3.** Procédé de préparation des sulfonylurées substituées de la formule générale I selon la revendication 1, caractérisé par le fait que l'on fait réagir un carbamate de formule IV

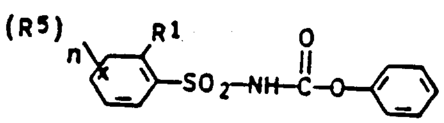

IV

de manière connue en soi, dans un solvant organique inerte, à 0 à 120° C, avec un 2-amino-hétéroaryléther III.

**4.** Procédé de préparation des sulfonylurées substituées de la formule générale I selon la revendication 1, caractérisé par le fait l'on fait réagir un sulfonamide de formule V correspondant

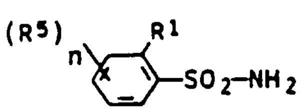

V

de manière connue en soi, dans un solvant organique inerte avec un phénylcarbamate VI

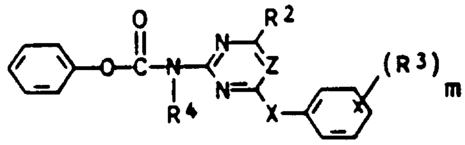

VI

5. Agent herbicide contenant une sulfonylurée substituée de la formule générale I selon la revendication 1 ou son sel ainsi que des auxiliaires de formulation usuels.

6. Procédé de lutte contre la croissance des plantes indésirables, caractérisé par le fait que l'on fait agir une quantité à effet herbicide d'une sulfonylurée substituée de la formule générale I selon la revendication 1 ou un de ses sels sur les plantes et/ou leur biotope.

7. Utilisation des sulfonylurées substituées de la formule générale I selon la revendication 1 comme agent herbicide.

8. Moyen pour influer sur la croissance des plantes contenant une sulfonylurée substituée de la formule générale I selon la revendication 1 ou son sel ainsi que les auxiliaires de formulation usuels.

9. Procédé pour influer sur la croissance des plantes, caractérisé par le fait qu'on fait agir une quantité régularisant la croissance d'une sulfonylurée substituée de la formule générale I ou un de ses sels sur les plantes et/ou leur biotope.

**Revendications pour l'Etat contractant suivant : ES**

1. Procédé de préparation de sulfonylurées substituées de la formule générale I

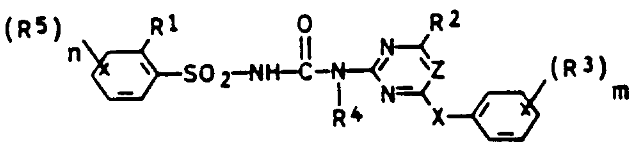

I

dans laquelle les substituants et les indices ont les significations suivantes :

X représente oxygène ou soufre

Z azote ou un groupe méthine = CH-

$R^1$ halogène, alcoxy carbonyle en C1-C4 qui peut porter un à trois des restes suivants : halogène, alcoxy en C1-C4, halogénoalcoxy en C1-C4, alkylthio en C1-C4 et/ou halogénoalkylthio en C1-C4, alcoxy en C1-C3, qui peut porter un à trois des restes suivants : halogène, alcoxy en C1-C4, halogénalcoxy en C1-C4, alkylthio en C1-C4 et/ou halogénalkylthio en C1-C4;

ou un reste $-CONR^6R^7$, où

$R^6$ hydrogène, un groupe alkyle en C1-C8 ou un groupe alcoxy en C1-C6 et

$R^7$ hydrogène ou un groupe alkyle en C1-C8;

$R^2$ halogène, alcoxy en C1-C4 ou alkyle en C1-C4 qui peuvent porter un à trois des restes suivants : halogène, alcoxy en C1-C4 et/ou alkylthio en C1-C4

$R^3$ alkyle en C1-C4 alcoxy en C1-C4, alkylthio en C1-C4, halogénalkyle en C1-C4, halogénalcoxy en C1-C4, halogénalkylthio en C1-C4, halogène, cyano, nitro, amino, mono-alkyle en C1-C4-amino, di-alkyle en C1-C4-amino, alcényle en C2-C6, alcoxycarbonyle en C1-C4, alcanoyle en C1-C4 ou benzyle;

un hétérocycle saturé, lié par son atome d'azote, à 5 ou 6 chainons, contenant outre des groupes méthylène un atome d'azote, ce cycle pouvant en outre contenir un atome d'oxygène ou de soufre

ainsi que -OCRR'O- liés sur des positions voisines du noyau, R et R' signifiant hydrogène ou alkyle en C1-C4

$R^4$ hydrogène ou alkyle en C1-C4

$R^5$ halogène, alkyle en C1-C4, alcoxy en C1-C4, alkylthio en C1-C4 ou halogénalkyle en C1-C4;
m 0 à 3 ou 0 à 5 quand R3 représente halogène les restes $R^3$ pouvant être différent quand m représente 2 ou 3
n 0 à 2, les restes $R^5$ pouvant être différents quand n = 2

ainsi que leurs sels acceptables pour l'environnement, caractérisé par le fait que l'on fait réagir un sulfonylisocyanate II

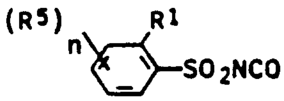

II

de manière connue en soi, dans un solvant organique inerte, avec un dérivé de 2-amino-hétéroaryléther III

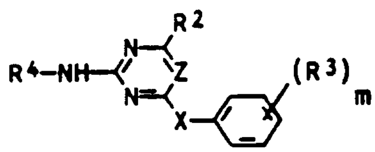

III

**2.** Procédé de préparation de sulfonylurées substituées de la formule générale I selon la revendication 1, caractérisé par le fait que l'on fait réagir un carbamate de formule IV

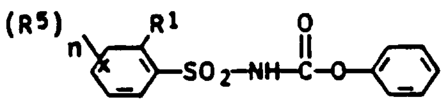

IV

de manière connue en soi, dans un solvant organique inerte, à 0 à 120° C, avec un 2-amino-hétéroaryléther III.

**3.** Procédé de préparation de sulfonylurées substituées de la formule générale I selon la revendication 1, caractérisé par le fait que l'on fait réagir un sulfonamide de formule V correspondant

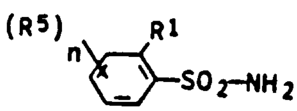

V

de manière connue en soi, dans un solvant organique inerte avec un phénylcarbamate VI

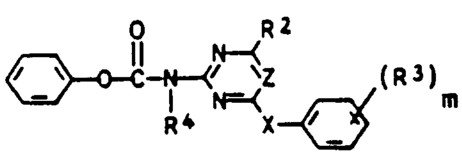

VI

**4.** Agent herbicide contenant une sulfonylurée substituée de la formule générale I selon la revendication 1 ou son sel ainsi que des auxiliaires de formulation usuels.

5. Procédé de lutte contre la croissance indésirable des plantes, caractérisé par le fait que l'on fait agir une quantité efficace comme herbicide d'une sulfonylurée substituée de la formule générale I selon la revendication 1 ou un de ses sels sur les plantes et/ou leur biotope.

6. Utilisation des sulfonylurées substituées de la formule générale I selon la revendication 1 comme agent herbicide.

7. Moyen pour influer sur la croissance des plantes contenant une sulfonylurée substituée de la formule générale I selon la revendication 1 ou son sel ainsi que les auxiliaires de formulation usuels.

8. Procédé pour influer sur la croissance des plantes, caractérisé par que le fait que l'on fait agir une quantité régularisant la croissance d'une sulfonylurée substituée de la formule générale I ou un de ses sels sur les plantes et/ou leur biotope.